# EUROPÄISCHE PATENTANMELDUNG

(11) **EP 1 818 414 A2**
(43) Veröffentlichungstag der Anmeldung: **15.08.2007**
(21) Anmeldenummer: 06077160.7
(22) Anmeldetag: 08.03.2002
(51) Int. Cl.: C12Q 1/68

(54) **Verfahren zum Nachweis von Cytosin-Methylierungsmustern mit hoher Sensitivität**

(30) Priorität: 09.03.2001 DE 10112515; 19.11.2001 DE 10158283
(62) Teilanmeldung aus: 02722194.4
(71) Anmelder: Epigenomics AG, 10178 Berlin (DE)
(72) Erfinder: Olek, Alexander, Dr., 12207 Berlin (DE); Berlin, Kurt, Dr., 14532 Stahnsdorf (DE)
(74) Vertreter: Schubert, Klemens

(57) **Zusammenfassung**

Die vorliegende Erfindung betrifft ein Verfahren zum Nachweis von Cytosin-Methylierung in DNA-Proben, wobei man die folgenden Schritte ausführt:
man behandelt eine genomische DNA-Probe, welche zu untersuchende DNA und Hintergrund-DNA DNA umfasst, chemisch derart, dass alle nicht methylierten Cytosinbasen in Uracil umgewandelt werden, während die 5-Methylcytosinbasen unverändert bleiben,
man amplifiziert die chemisch behandelte DNA-Probe unter Verwendung von mindestens 2 Primeroligonukleotiden sowie einer Polymerase, wobei die zu untersuchende DNA gegenüber der Hintergrund-DNA als Templat bevorzugt wird und
man analysiert die Amplifikate und schließt aus dem Vorliegen eines Amplifikates und/oder aus der Analyse weiterer Positionen auf den Methylierungsstatus in der zu untersuchenden DNA.

SEQUENCE LISTING

<110>  Epigenomics AG

<120>  Verfahren zum Nachweis von Cytosin-Methylierungsmustern
mit hoher Sensitivität

<130>  E01/1289/EP

<160>  43

<170>  PatentIn version 3.3

<210>  1
<211>  24
<212>  DNA
<213>  Artificial Sequence

<220>
<223>  Primer

<400>  1
gctctatggt cttgtctaac cgta                                          24

<210>  2
<211>  18
<212>  DNA
<213>  Artificial Sequence

<220>
<223>  Primer

<400>  2
aggtggtggt ggcggtgg                                                 18

<210> 3
<211> 28
<212> DNA
<213> Artificial Sequence

<220>
<223> Primer

<400> 3
atggttttgt ttaatygtag agttgttt 28

<210> 4
<211> 27
<212> DNA
<213> Artificial Sequence

<220>
<223> Oligonukleotid

<400> 4
taaacccraa aaaaaaaac ccaatat 27

<210> 5
<211> 18
<212> DNA
<213> Artificial Sequence

<220>
<223> Oligonukleotid

<400> 5
ctactcaacg aaaacaaa 18

<210>  6
<211>  18
<212>  DNA
<213>  Artificial Sequence

<220>
<223>  Oligonukleotid

<400>  6
ctactcaaca aaaacaaa                                                    18

<210>  7
<211>  28
<212>  DNA
<213>  Artificial Sequence

<220>
<223>  Primer

<400>  7
taagtatgtt gaagaaagat tattgtag                                         28

<210>  8
<211>  28
<212>  DNA
<213>  Artificial Sequence

<220>
<223>  Primer

<400>  8
taaaaactat cccataataa ctcccaac                                         28

<210> 9
<211> 21
<212> DNA
<213> Artificial Sequence

<220>
<223> Primer

<400> 9
taagtatgtt gaagaaagat t 21

<210> 10
<211> 21
<212> DNA
<213> Artificial Sequence

<220>
<223> Primer

<400> 10
aatccccata aacttaccaa a 21

<210> 11
<211> 18
<212> DNA
<213> Artificial Sequence

<220>
<223> Primer

<400> 11
ttatgtgaat tttgaaag 18

<210>  12
<211>  19
<212>  DNA
<213>  Artificial Sequence

<220>
<223>  Primer

<400>  12
ggaaagaggg aaaggtttt                                                    19

<210>  13
<211>  22
<212>  DNA
<213>  Artificial Sequence

<220>
<223>  Primer

<400>  13
tactaaaaac tctaaacccc at                                                22

<210>  14
<211>  20
<212>  DNA
<213>  Artificial Sequence

<220>
<223>  Primer

<400>  14
gttttctgtt attagtgagt                                                   20

<210> 15
<211> 18
<212> DNA
<213> Artificial Sequence

<220>
<223> Primer

<400> 15
tcctaaatcc cctaaacc 18

<210> 16
<211> 21
<212> DNA
<213> Artificial Sequence

<220>
<223> Blocker-Oligonukleotid

<220>
<221> modified_base
<222> (21)..(21)
<223> mit Phosphat-Gruppe modifiziertes Thymin

<400> 16
gtgagtatgt gtggtttgtg t 21

<210> 17
<211> 22
<212> DNA
<213> Artificial Sequence

<220>

<223>  Blocker-Oligonukleotid

<220>

<221>  modified_base

<222>  (21)..(21)

<223>  Mit Phospha-Gruppe modifiziertes Adenin

<400>  17

taaaccccca tcccaaatct ca                                              22

<210>  18

<211>  22

<212>  DNA

<213>  Artificial Sequence

<220>

<223>  Blocker-Oligonukleotid

<220>

<221>  modified_base

<222>  (22)..(22)

<223>  Mit Phosphatgruppe modifiziertes Thymin

<400>  18

gtgagtatgt gtggtttgtg tt                                              22

<210>  19

<211>  27

<212>  DNA

<213>  Artificial Sequence

<220>
<223> Anker-Oligonukleotid GSTp1-Fluo

<220>
<221> modified_base
<222> (27)..(27)
<223> Mit Fluorescein modifiziertes Thymin

<400> 19
tttagagttt ttagtatggg gttaatt 27

<210> 20
<211> 22
<212> DNA
<213> Artificial Sequence

<220>
<223> Hybridisierungssonde GSTp1-Red 705

<220>
<221> modified_base
<222> (1)..(1)
<223> Mit Red 707 modifiziertes Guanin

<220>
<221> modified_base
<222> (22)..(22)
<223> Mit Phosphat modifiziertes Thymin

<400> 20
gtattaggtt tgggtttttg gt 22

<210>  21

<211>  23

<212>  DNA

<213>  Artificial Sequence

<220>

<223>  Hybridisierungssonde GSTp1-Red 650

<220>

<221>  modified_base

<222>  (1)..(1)

<223>  Mit Red 650 modifiziertes Thymin

<220>

<221>  modified_base

<222>  (23)..(23)

<223>  Mit Phosphat modifiziertes Guanin

<400>  21

tagtattagg ttcgggtttt cgg                                              23

<210>  22

<211>  21

<212>  DNA

<213>  Artificial Sequence

<220>

<223>  Primer

<400>  22

gttttctgtt attagtgagt a                                                21

<210>  23

<211>  21
<212>  DNA
<213>  Artificial Sequence

<220>
<223>  Blocker-Oligonukleotid

<220>
<221>  modified_base
<222>  (21)..(21)
<223>  Mit Phosphat modifiziertes Cytosin

<400>  23
taaaccccca tcccaaatct c                                                21

<210>  24
<211>  35
<212>  DNA
<213>  Artificial Sequence

<220>
<223>  TaqMan-Sonde

<220>
<221>  modified_base
<222>  (1)..(1)
<223>  Mit Black hole modifiziertes Thymin

<220>
<221>  modified_base
<222>  (35)..(35)
<223>  Mit FAM modifiziertes Guanin

<400> 24
taattcgtag tattaggttc gggttttcgg taggg 35

<210> 25
<211> 66
<212> DNA
<213> Artificial Sequence

<220>
<223> Mit Bisulfit behandelte DNA

<400> 25
tcgtcgtcgt agttttcgtt attagtgagt acgcgcggtt cgcgttttcg
gggatggggt 60

ttagag 66

<210> 26
<211> 66
<212> DNA
<213> Artificial Sequence

<220>
<223> Mit Bisulfit behandelte DNA

<400> 26
ttgttgttgt agtttttgtt attagtgagt atgtgtggtt tgtgtttttg
gggatggggt 60

ttagag 66

<210> 27
<211> 15

<212>  DNA
<213>  Artificial Sequence

<220>
<223>  Primer

<400>  27
atcactcatg cgcgc                                                        15

<210>  28
<211>  15
<212>  DNA
<213>  Artificial Sequence

<220>
<223>  Primer

<400>  28
atcactcatr crcrc                                                        15

<210>  29
<211>  15
<212>  DNA
<213>  Artificial Sequence

<220>
<223>  Primer

<220>
<221>  misc_feature
<222>  (10)..(10)
<223>  Inosin

<220>
<221> misc_feature
<222> (12)..(12)
<223> Inosin

<220>
<221> misc_feature
<222> (14)..(14)
<223> Inosin

<400> 29
atcactcatn cncnc 15

<210> 30
<211> 13
<212> DNA
<213> Artificial Sequence

<220>
<223> Primer

<400> 30
aataatcact cat 13

<210> 31
<211> 16
<212> DNA
<213> Artificial Sequence

<220>
<223> Blocker-Oligonukleotid

<400> 31
acacaccaaa cacaaa 16

<210>  32
<211>  17
<212>  DNA
<213>  Artificial Sequence

<220>
<223>  Blocker-Oligonukleotid

<400>  32
agtgagtaac acaccaa                                                    17

<210>  33
<211>  18
<212>  DNA
<213>  Artificial Sequence

<220>
<223>  Blocker-Oligonukleotid

<400>  33
atcactcata gagagcaa                                                   18

<210>  34
<211>  18
<212>  DNA
<213>  Artificial Sequence

<220>
<223>  Primer

<400>  34
atcactcatr crcrccaa                                                   18

<210>  35
<211>  18
<212>  DNA
<213>  Artificial Sequence

<220>
<223>  Blocker-Oligonukleotid

<400>  35
acacaccaaa cacaaaaa                                                    18

<210>  36
<211>  19
<212>  DNA
<213>  Artificial Sequence

<220>
<223>  Blocker-Oligonukleotid

<400>  36
acacaccaaa cacaaaaac                                                   19

<210>  37
<211>  14
<212>  DNA
<213>  Artificial Sequence

<220>
<223>  Blocker-Oligonukleotid

<400>  37
acaccaaaca caaa                                                        14

<210> 38
<211> 13
<212> DNA
<213> Artificial Sequence

<220>
<223> Primer

<400> 38
cccctacccc aaa 13

<210> 39
<211> 153
<212> DNA
<213> Artificial Sequence

<220>
<223> Amplifikationsprodukt eines 153 bp GSTp1-Fragments (Po-
sition 1242-1393 in Sequenz AccNr M24485.1 mit Bisulfit-DNA
spezifischen Primern

<400> 39
gtttttgtta ttagtgagta tgtgtggttt gtgtttttgg ggatggggtt ta-
gagttttt 60

agtatggggt taatttgtag tattaggttt gggtttttgg tagggttttt
tgtttatttt 120

gagatttggg atgggggttt aggggattta gga 153

<210> 40
<211> 153

<212>  DNA
<213>  Artificial Sequence

<220>
<223>  Mit SssI-behandeltes DNA-Fragment

<400>  40
gttttcgtta ttagtgagta cgcgcggttc gcgttttcgg ggatggggtt ta-
gagttttt                                                          60

agtatggggt taattcgtag tattaggttc gggttttcgg tagggttttt
cgtttatttc                                                        120

gagattcggg acgggggttt aggggattta gga                              153

<210>  41
<211>  19
<212>  DNA
<213>  Artificial Sequence

<220>
<223>  Primer

<400>  41
gttttrgtta ttagtgagt                                              19

<210>  42
<211>  22
<212>  DNA
<213>  Artificial Sequence

<220>
<223>  Blocker-Oligonukleotid

<220>

<221>  modified_base

<222>  (1)..(1)

<223>  Mit Phosphat modifiziertes Adenin

<400>  42

actctaaacc ctacccccaa at                                              22

<210>  43

<211>  18

<212>  DNA

<213>  Artificial Sequence

<220>

<223>  Primer

<400>  43

ccaaatcccc taaatcct                                                   18

## Beschreibung

Die vorliegende Erfindung betrifft ein Verfahren zum Nachweis von Cytosin-Methylierung in DNA-Proben.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms. 5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neu und die mittlerweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällung- und Reinigungsschritte durch schnelle Dialyse ersetzt (Olek A, Oswald J, Walter J. A modified and improved method for bisulphate based cytosine methylation analysis. Nucleic Acids Res. 1996 DEC 15;24(24):5064-6). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein T, DePamphilis ML, Zorbas H. Identifying 5-methylcytosine and related modifications in DNA genomes. Nucleic Acids Res. 1998 May 15;26(10):2255-64.

Die Bisulfit-Technik wird bisher bis auf wenige Ausnahmen (z. B. Zeschnigk M, Lich C, Buiting K, Dörfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) nur in der Forschung angewendet. Immer aber werden kurze, spezifische Stücke eines bekannten Gens nach einer Bisulfit-Behandlung amplifiziert und entweder komplett sequenziert (Olek A, Walter J. The pre-implantation ontogeny of the H19 methylation imprint. Nat Genet. 1997 Nov.;17(3):275-6) oder einzelne Cytosin-Positionen durch eine "Primer-Extension-Reaktion" (Gonzalgo ML, Jones PA. Rapid quantitation of methylation differences at specific sites using methylation-sensitive single nucleotide primer extension (Ms-SNuPE). Nucleic Acids Res. 1997 Jun. 15;25(12):2529-31, WO-Patent 9500669) oder einen Enzymschnitt (Xiong Z, Laird PW. COBRA: a sensitive and quantitative DNA methylation assay. Nucleic Acids Res. 1997 Jun. 15;25(12):2532-4) nachgewiesen. Zudem ist auch der Nachweis durch Hybridisierung beschrieben worden (Olek et al., WO 99 28498).

Harnstoff verbessert die Effizienz der Bisulfit-Behandlung vor der Sequenzierung von 5-Methylcytosin in genomischer DNA (Paulin R, Grigg GW, Davey MW, Piper AA. Urea improves efficiency of bisulphate-mediated sequencing of 5'-methylcytosine in genomic DNA. Nucleic Acids Res. 1998 Nov. 1;26(21):5009-10).

Weitere Publikationen, die sich mit der Anwendung der Bisulfit-Technik zum Methylierungsnachweis bei einzelnen Genen befassen, sind:
Grigg G, Clark S. sequencing 5-methylcytosine residues in genomic DNA. Bioassays. 1994 Jun.;16(6):431-6, 431; Zeschnigk M, Schmitz B, Dittrich B, Buiting K, Horsthemke B, Dörfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol fort bisulphate genomic sequencing. Nucleic Acids Res. 1994 Feb. 25;22(4):695-6; Martin V, Ribieras S, Song-Wang X, Rio MC, Dante R. Genomic sequencing indicates a correlation between DNA hypomethylation in the 5' region of the pS2 gene andin its expression in human breast cancer cell lines. Gene. 1995 May 19;157(1-2):261-4; WO 97 46705, WO 95 15373 und WO 45560.

Ein weiteres bekanntes Verfahren ist die sogenannte methylierungssensitive PCR (Herman JG, Graff JR, Myohanen S, Nelkin BD, Baylin SB. (1996), Methylation-specific PCR: a novel PCR assay for methylation status of CpG islands. Proc Natl Acad Sci U S A. Sep 3;93(18):9821-6). Für dieses Verfahren werden Primer verwendet, die entweder nur an eine Sequenz hybridisieren, die durch die Bisulfit-Behandlung einer an der betreffenden Position unmethylierten DNA entsteht, oder aber umgekehrt Primer, welche nur an eine Nukleinsäure bindet, die durch die Bisulfit-Behandlung einer an der betreffenden Position unmethylierten DNA entsteht. Mit diesen Primer können demnach Amplifikate erzeugt werden, deren Detektion wiederum Hinweise auf das Vorliegen einer methylierten oder unmethylierten Position in der Probe liefern, an welche die Primer binden.

Ein neueres Verfahren ist auch der Nachweis von Cytosin-Methylierung mittels einer Taqman PCR, das als Methyl-Light bekannt geworden ist (WO00/70090). Mit diesem Verfahren ist es möglich, den Methylierungsstatus einzelner oder weniger Positionen direkt im Verlauf der PCR nachzuweisen, so dass sich eine nachfolgende Analyse der Produkte erübrigt.

Orum H. PCR clamping. Cur Issues Mol Biol, 2000 Jan; 2 (1): 27-30 und Yu et al. Specific inhibition of PCR by non-extendable oligonucleotides using a 5' to 3' exonucleasedeficient DNA Polymerase. Biotechniques, Eaton Publishing, US, 1997 Okt; 23 (4):716-20, beschreiben jeweils die Verwendung von Blockermolekülen um eine zielsequenzspezifische PCR Amplifikation zu erreichen.

Eine Übersicht über den Stand der Technik in der Oligomer Array Herstellung lässt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonucleotide bei vermindertem nichtspezifischen Hintergrundsignal entnehmen.

Für die Abtastung eines immobilisierten DNA-Arrays sind vielfach fluoresziert markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektrometrie (MALDI-TOF) ist eine sehr leistungsfähige Entwicklung für die Analyse von Biomolekülen (Karas M, Hillenkamp F. Laser desorption ionization of proteins with molecular masses exceeding 10,000 daltons. Anal Chem. 1988 Oct. 15;60(20):2299-301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektroskopie eignet sich ausgezeichnet zur Analyse von Peptiden und Proteinen. Die Analyse von Nukleinsäuren ist etwas schwieriger (Gut, I. G. und Beck, S. (1995), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlerweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis, Molecular Cloning: A Laboratory Manual, 1989.

Es sind demnach bislang vielerlei Verfahren zur Methylierungsanalyse Stand der Technik. Die vorliegende Erfindung soll jedoch das Problem lösen, dass die gängigen Verfahren nicht in der Lage sind, eine in einer Körperflüssigkeit oder Serum befindliche zu untersuchende DNA gezielt zu amplifizieren, wenn zugleich andere, sequenzhomologe DNA-Abschnitte anderen Ursprungs zugegen sind.

Die zu untersuchende DNA sowie die ansonsten vorhandenen, im folgenden Hintergrund-DNA genannten Nukleinsäuren, werden in aller Regel gleichermaßen amplifiziert, da die verwendeten Primer auch nicht in der Lage sind, zwischen zu untersuchender DNA und Hintergrund-DNA zu unterscheiden. Eine Möglichkeit zur Unterscheidung dieser DNAs ergibt sich jedoch durch das unterschiedliche Methylierungsmuster. Ein gängiges Verfahren ist die methylierungssensitive PCR, kurz MSP (Herman JG, Graff JR, Myohanen S, Nelkin BD, Baylin SB. (1996), Methylation-specific PCR: a novel PCR assay for methylation status of CpG islands. Proc Natl Acad Sci U S A. Sep 3;93(18):9821-6). Dieses Verfahren besteht aus mehreren Teilschritten. Zunächst wird eine dem Stand der Technik entsprechende Bisulfit-Behandlung durchgeführt, welche wiederum dazu führt, dass alle Cytosinbasen in Uracil umgewandelt werden, während die methylierten Cytosinbasen (5-Methylcytosin) unverändert bleiben. Im nächsten Schritt verwendet man nun Primer, welche vollständig komplementär zu einer methylierten, mit Bisulfit umgewandelten DNA sind, nicht jedoch zu einer entsprechenden DNA welche ursprünglich nicht methyliert vorlag. Das führt bei der Durchführung einer PCR mit einem solchen Primer dazu, dass ausschließlich die ursprünglich methylierte DNA amplifiziert wird. Entsprechend ist es möglich, einen Primer zu verwenden, der im Gegenzug nur die unmethylierte DNA amplifiziert. Auf diese Art und Weise können, wenn zu analysierende DNA sowie Hintergrund DNA zugegen sind, ausschließlich die zu untersuchenden DNA Fragmente selektiv erzeugt werden, sofern sich diese hinsichtlich ihres Methylierungsstatus in einer CpG Position von der Hintergrund DNA unterscheiden. Stand der Technik ist es nun, aus dem Nachweis eines solchen zu untersuchenden DNA-Moleküls auf den Methylierungszustand oder das Vorliegen einer zu untersuchenden DNA rückzuschließen, was wiederum eine Diagnose beispielsweise einer Tumorerkrankung in Patienten prinzipiell erlaubt, da es bekannt ist, das beispielsweise die Serum DNA-Konzentration sich in Tumorpatienten zum Teil drastisch erhöht. Nur die von den Tumoren stammende DNA soll dann neben der Hintergrund-DNA nachgewiesen werden. Prinzipiell vergleichbar ist die Analyse von DNA in anderen Körperflüssigkeiten.

Das hier geschilderte Verfahren, was als der nächstliegende Stand der Technik zu betrachten ist, hat jedoch einige Nachteile. Es ist beispielsweise nicht möglich, aus der Nachweisbarkeit eines amplifizierten Fragmentes zu untersuchender DNA auf die im Serum vorhandene Menge zu schließen. Schon geringste Mengen solcher DNA reichen aus, um ein positives Resultat zu erzielen, was zum einen ein Vorteil ist, sich aber auch sehr nachteilig auswirken kann, wenn man zum Beispiel den Effekt einer Tumorresektion auf die Serum DNA beurteilen will. Der größte Nachteil ist jedoch, dass es viele Methylierungspositionen gibt, in welchen sich zu untersuchende DNA und Hintergrund-DNA nur graduell unterscheiden. Es ist offensichtlich, dass das existierenden MSP Verfahren nur dann durchgeführt werden kann, wenn man weiß, dass sich die Hintergund-DNA von der zu untersuchenden DNA in der betreffenden CpG Position definitiv und zu 100% unterscheidet, will man nicht falsche positive Ergebnisse riskieren. Ist es dagegen in einem Tumorgewebe typisch, dass in z. B. 95 % der Tumorzellen eine bestimmte Position methyliert vorliegt, in der ansonsten vorhandenen Hintergrund-DNA jedoch nur maximal 5% methyliert vorliegen, so ist es mit der MSP Methode nicht möglich, aussagekräftige Ergebnisse zu produzieren, da eine Quantifizierung der Templat-DNA mittels PCR prinzipiell nicht oder nur mit erhöhtem Aufwand möglich ist. Zudem liegt dieser Erfindung die Erkenntnis zugrunde, dass es oft Muster von Methylierungszuständen in einem DNA-Fragment sind, die typisch für einen bestimmten Typ von Zellen, zum Beispiel einer Tumorzelle, sind.

Stand der Technik ist wiederum ein von Epigenomics entwickeltes Verfahren, welches zu untersuchende DNA und Hintergrund-DNA nach Bisulfit-Behandlung gleichermaßen amplifiziert und dann die im Fragment enthaltenen ehemaligen CpG Positionen durch Hybridisierungstechniken untersucht, alternativ mittels MiniSequenzierung oder anderen gängigen Verfahren. Dies hat den Vorteil, dass man ein quantitatives Bild bezüglich der untersuchten Methylierungspositionen erhält, d. h. es erfolgt die Bestimmung des Methylierungsgrades einer Vielzahl von Positionen, was z. B. bei soliden Tumoren eine sehr genau Klassifizierung ermöglicht. Der Nachteil dieser Methode ist jedoch, dass sie in den Fällen, in denen die Hintergrund-DNA stark überwiegt, keine genau Aussage liefern kann, da diese ja genau wie die zu untersuchende DNA amplifiziert wird und beide im Gemisch analysiert werden. Dieses Problem existiert nicht bei der Analyse von soliden Tumoren, wo man das zu untersuchende Material gezielt auswählen kann, es kann jedoch die Analyse von beispielsweise Serum-DNA erschweren.

Ziel der vorliegenden Erfindung ist es nun, die Nachteile des Standes der Technik zu überwinden und die Vorteile beider Verfahren für die Detektion von Körperflüssigkeiten und Serum zu kombinieren.

Die Aufgabe wird dadurch gelöst, dass ein Verfahren zum Nachweis von Cytosin-Methylierung in DNA-Proben geschaffen wird bei dem dass man die folgenden Schritte ausführt:
man behandelt eine genomische DNA-Probe, welche zu untersuchende DNA und Hintergrund-DNA DNA umfasst, chemisch derart, dass alle nicht methylierten Cytosinbasen in Uracil umgewandelt werden, während die 5-Methylcytosinbasen unverändert bleiben,
man amplifiziert die chemisch behandelte DNA-Probe unter Verwendung von mindestens 2 Primeroligonukleotiden sowie einer Polymerase, wobei die zu untersuchende DNA gegenüber der Hintergrund-DNA als Templat bevorzugt wird und
man analysiert die Amplifikate und schließt aus dem Vorliegen eines Amplifikates und/oder aus der Analyse weiterer Positionen auf den Methylierungsstatus in der zu untersuchenden DNA.

Erfindungsgemäß bevorzugt ist es, dass man die Proben DNA aus Serum oder anderen Körperflüssigkeiten eines Individuums gewinnt.

Es ist weiterhin erfindungsgemäß bevorzugt, dass man die Proben DNA aus Zellinien, Blut, Sputum, Stuhl, Urin, Serum, Gehirn-Rückenmarks-Flüssigkeit, in Paraffin einbettetem Gewebe, beispielsweise Gewebe von Augen, Darm, Niere, Hirn, Herz, Prostata, Lunge, Brust oder Leber, histologischen Objektträgern und allen möglichen Kombinationen hiervon gewinnt.

Es ist ganz besonders erfindungsgemäß bevorzugt, dass man die chemische Behandlung mit einem Bisulfit (=Disulfit, Hydrogensulfit) durchführt. Bevorzugt ist es auch, dass die chemische Behandlung nach Einbetten der DNA in Agarose erfolgt. Es ist auch und weiterhin bevorzugt, dass bei der chemischen Behandlung ein die DNA-Duplex denaturierendes Reagenz und/oder ein Radikalfänger zugegen ist.

Bevorzugt ist es, dass man die Amplifikation im zweiten Schritt in Gegenwart mindestens eines weiteren Oligonukleotids durchführt, welches an ein 5'-CG-3'-Dinukleotid oder ein 5'-TG-3'-Dinukleotid oder ein 5'-CA-3'-Dinukleotid bindet, wobei das weitere Oligonukleotid bevorzugt an die Hintergrund-DNA bindet und deren Amplifikation beeinträchtigt.

Besonders bevorzugt ist es, dass diese Bindungsstelle des weiteren Oligonukleotids oder PNA-Oligomers mit den Bindungstellen der Primer auf der Hintergund-DNA überlappt und das weitere Oligonukleotid das Binden mindestens eines Primeroligonukleotids an die Hintergrund-DNA behindert.

Es ist wiederum besonders bevorzugt, dass mindestens zwei weitere Olgonukleotide oder PNA-Oligomere eingesetzt werden, wobei deren Bindungsstelle wiederum jeweils mit der Bindungsstelle eines Primers an die Hintergund-DNA überlappt und die weiteren Oligonukleotide und/oder PNA-Oligomere das Binden beider Primeroligonukleotide an die Hintergrund-DNA behindern.

Zudem ist es besonders bevorzugt, dass jeweils eines der weiteren Oligonukleotide und/oder PNA-Oligomere das Binden des Forward-Primers behindert während das jeweils andere das Binden des Reverse-Primers behindert.

Besonders bevorzugt ist es, dass die weiteren Oligonukleotide und/oder PNA-Oligomere in mindestens der fünffachen Konzentration im Vergleich zu den Primeroligonuleotide vorliegen.

In einer weiteren besonders bevorzugten Verfahrensvariante binden die weiteren Oligonukleotide und/oder PNA-Oligomere an die Hintergrund-DNA und behindern damit die vollständige Primeroligonukleotid-verlängerung in der Polymerasereaktion. Dabei ist es wiederum besonders, dass die verwendete Polymerase keine 5'-3' -Exonukleaseaktivität aufweist. Eine weitere bevorzugte Variante ist es, dass die weiteren Oligonukleotide am 5'-Ende modifiziert vorliegen und damit von einer Polymerase mit 5'-3'-Exonuklease-aktivität nicht signifikant abgebaut werden können.

Weiterhin ist es erfindungsgemäß bevorzugt, dass man die chemisch behandelte DNA-Probe im zweiten Schritt unter Verwendung von mindestens 2 Primeroligonukleotiden und einem weiteren Oligonukleotid, welches an ein 5'-CG-3'-Dinukleotid oder ein 5'-TG-3'-Dinukleotid oder ein 5'-CA-3'-Dinukleotid hybridisiert, und mindestens einem Reporteroligonukleotid, welches an ein 5'-CG-3'-Dinukleotid oder ein 5'-TG-3'-Dinukleotid oder ein 5'-CA-3'-Dinukleotid hybridisiert, sowie einer Polymerase amplifiziert; wobei das weitere Oligonukleotid bevorzugt an die Hintergrund-DNA bindet und deren Amplifikation beeinträchtigt, und wobei das Reporteroligonukleotid bevorzugt an die zu untersuchende DNA bindet und deren Amplifikation anzeigt. Dabei ist es vorteilhaft, dass man zusätzlich zu dem Reporteroligonukleotid ein weiteres mit einem Fluoreszenzfarbstoff markiertes Oligomer verwendet, welches unmittelbar benachbart zu dem Reporteroligonukleotid hybridisiert und sich diese Hybridisierung mittels Fluoreszenz Resonanz Energietransfer nachweisen lässt. Weiterhin vorteilhaft ist es, dass ein Taqman-Assay durchgeführt wird. Bevorzugt ist es auch, dass ein Lightcycler Assay durchgeführt wird.

Es ist ferner erfindungsgemäß bevorzugt, dass die zusätzlich zu den Primern verwendeten Oligonukleotide nicht über eine 3'-OH Funktion verfügen. Weiterhin ist bevorzugt, dass die Reporteroligonukleotide mindestens eine Fluoreszenzmarkierung tragen. Ferner ist auch bevorzugt, dass die Reportermoleküle die Amplifikation entweder durch eine Zunahme oder eine Abnahme der Fluoreszenz anzeigen. Dabei ist es besonders vorteilhaft, dass man die Zunahme oder Abnahme der Fluoreszenz auch direkt zur Analyse verwendet und aus dem Fluorenzenzsignal auf einen Methylierungszustand der zu analysierenden DNA schließt.

Bevorzugt ist es erfindungsgemäß ferner, dass die Hintergrund-DNA in 100 facher Konzentration im Vergleich zur zu untersuchenden DNA vorliegt. Weiterhin ist bevorzugt, dass die Hintergrund-DNA in 1000 facher Konzentration im Vergleich zur zu untersuchenden DNA vorliegt.

Bevorzugt ist es ferner, dass die Analyse, oder gegebenenfalls die weitere Analyse mittels Hybridisierung an Oligomer-Arrays erfolgt, wobei Oligomere Nukleinsäuren oder in ihren Hybridisierungseigenschaften ähnliche Moleküle wie PNAs sein können.

Vorteilhaft ist es erfindungsgemäß auch, dass die Oligomere über einen 12-22 Basen langen Abschnitt an die zu analysierende DNA hybridisieren und sie ein CG, TG oder CA Dinukleotid umfassen.

Es ist bevorzugt, dass der Methylierungsstatus von mehr als 20 Methylierungspositionen der zu analysierenden DNA in einem Experiment nachgewiesen wird.

Weiterhin ist bevorzugt, dass der Methylierungsstatus von mehr als 60 Methylierungspositionen der zu analysierenden DNA in einem Experiment nachgewiesen wird.

Auch ist es erfindungsgemäß bevorzugt, dass die Analyse, oder gegebenenfalls die weitere Analyse durch Längenmessung der amplifizierten zu untersuchenden DNA erfolgt, wobei Methoden zur Längenmessung Gelelektrophorese, Kapillargelelektrophorese, Chromatographie (z.B. HPLC), Massenspektrometrie und andere geeignete Methoden umfassen. Vorteilhaft ist es dabei auch, dass Methoden zur Sequenzierung die Sanger-Methode, Maxam-Gilbert-Methode und andere Methoden wie Sequencing by Hybridisation (SBH) umfassen.

Erfindungsgemäß bevorzugt ist auch ein Verfahren, wobei man die Sequenzierung für jede oder eine kleine Gruppe von CpG Positionen mit jeweils einem separaten Primeroligonukleotid ausführt und die Verlängerung der Primer nur eine oder einige wenige Basen ausmacht, und man aus der Art der Primerverlängerung auf den Methylierungsstatus der betreffenden Positionen in der zu untersuchenden DNA schließt.

Weiterhin ist bevorzugt, dass man aus dem Methylierungsgrad an den verschiedenen untersuchten CpG Positionen auf das Vorliegen einer Erkankung oder eines anderen medizinischen Zustandes des Patienten schließt.

Vorteilhaft ist es, dass die Amplifikate selbst für die Detektion mit einer nachweisbaren Markierung versehen sind. Weiterhin vorteilhaft ist es, dass die Markierungen Fluoreszenzmarkierungen sind. oder/und dass die Markierungen Radionuklide sind oder/und dass die Markierungen ablösbare Massenmarkierungen sind, die in einem Massenspektrometer nachgewiesen werden.

Bevorzugt ist ferner, dass bei der Amplifikation einer der Primer an eine Festphase gebunden ist.

Erfindungsgemäß ist es auch, dass die Amplifikate insgesamt im Massenspektrometer nachgewiesen werden und somit durch ihre Masse eindeutig charakterisiert sind.

Ein weiterer Gegenstand der vorliegenden Erfindung ist auch die Verwendung eines erfindungsgemäßen Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Vorteilhaft ist dabei die Verwendung eines erfindungsgemäßen Verfahrens zur Unterscheidung von Zelltypen oder Geweben oder zur Untersuchung der Zelldifferenzierung.

Gegenstand der vorliegenden Erfindung ist auch ein Kit, bestehend aus einem Bisulfit enthaltenen Reagenz, Primern und weiteren Oligonuleotiden ohne 3'-OH Funktion zur Herstellung der Amplifikate, sowie optional einer Anleitung zur Durchführung eines erfindungsgemäßen Assays

Die vorliegende Erfindung beschreibt somit ein Verfahren zur Detektion des Methylierungszustandes genomischer DNA Proben. Im Gegensatz zu bislang bekannten Verfahren wird der Methylierungsgrad eines Satzes von CpG Positionen in einer ausgewählten Untergruppe von DNA-Fragmenten z. B. in Serum bestimmt, so dass eine Analyse auch in Gegenwart eines Überschusses an diagnostisch nicht relevanter Hintergrund-DNA möglich ist.

Dabei besteht das bevorzugte Verfahren wiederum aus mehreren Schritten, die sich wie folgt zusammenfassen lassen:
Zuerst werden dem Patienten Serum und/oder andere Körperflüssigkeiten entnommen und die darin befindliche DNA wenn erforderlich isoliert. Anschliessend wird im zweiten Schritt eine chemische Behandlung, bevorzugt mit einem Bisulfit (=Hydrogensulfit, Disulfit) durchgeführt, wobei beispielsweise alle nicht methylierten Cytosinbasen in Uracil umgewandelt werden, die methylierten Cytosinbasen (5-Methylcytosin) jedoch unverändert bleiben. Im dritten Verfahrensschritt wird nun eine Amplifikation durchgeführt, bei der bevorzugt die zu untersuchende DNA amplifiziert wird, nicht aber oder nur in geringerem Maße die Hintergrund-DNA. Im folgenden, vierten Schritt werden nun die amplifizierten Fragmente auf Ihre Methlylierungssignatur hin analysiert und der Methylierungsgrad mehrerer ehemaliger CpG Positionen in den Amplifikaten bestimmt. Im fünften Verfahrensschritt wird aus dem Methylierungsgrad an den verschiedenen untersuchten CpG Positionen auf das Vorliegen einer Erkankung oder eines anderen medizinischen Zustandes des Patienten geschlossen.

Das Wesen der vorliegenden Erfindung ist es nun, dass zwei Arten von CpG Positionen eine Rolle spielen und gleichermaßen zur Analyse beitragen und die nachfolgend als "Qualifier"-Positionen und "Classifier"-Positionen benannt werden sollen. Die Qualifier-Positionen dienen dazu, in der Amplifikation die zu analysierende DNA von der Hintergrund-DNA zu unterscheiden. Dies kann technisch, wie im folgenden detailliert dargelegt, auf unterschiedliche Art und Weise erfolgen. Eigenschaft dieser Positionen ist es jedoch, dass ihr Methylierungsgrad in zu untersuchender DNA möglichst verschieden ist von dem in der Hintergrund-DNA und die zu einer Bevorzugung der zu untersuchenden DNA in der Amplifikation führt. Die Classifier-Positionen dienen im Gegensatz dazu, aus dem Amplifikat, erzeugt überwiegend aus der zu untersuchenden DNA, über den jeweiligen Methlierungsgrad die für die Diagnose bedeutende Information zu extrahieren. Es können bis zu einigen 100 solcher Classifier Positionen für eine Analyse verwendet werden, erfolgt die Analyse beipielsweise auf Oligomer-Arrays, auch wenn dies oftmals nicht erforderlich sein wird. Es ist jedoch in diesem Fall nicht das Entstehen eines bestimmten Amplifikates, das für das Untersuchungsergebnis von Bedeutung ist, sondern vielmehr die Analyse der CpG-Positionen in selbigem Amplifikat. In einigen Fällen ist es jedoch sicher möglich und sinnvoll, die aus dem Entstehen eines Amplifikates abzuleitende Information in die Analyse mit einzubeziehen, in diesem Fall sind einige Positionen dann zugleich Classifier und Qualifier.

Der erste Schritt des Verfahrens, die Gewinnung von Proben, erfolgt bevorzugt durch Entnahme von Köperflüssigkeiten wie z. B. Sputum oder aber Serum, jedoch ist es offenkundig dass das Verfahren mit vielerlei Proben aus unterschiedlichen Quellen durchführbar ist, die hier ohne Anspruch auf Vollständigkeit aufgeführt sind.

Bevorzugt wird die in dem Verfahren eingesetzte genomische DNA aus einer DNA-Probe erhalten, wobei Quellen für DNA z. B. Zellinien, Blut, Sputum, Stuhl, Urin, Serum, Gehirn-Rückenmarks-Flüssigkeit, in Paraffin eingebettetes Gewebe, beispielsweise Gewebe von Augen, Darm, Niere, Hirn, Herz, Prostata, Lunge, Brust oder Leber, histologische Objektträger und alle möglichen Kombinationen hiervon umfassen.

Es erfolgt in einigen Fällen vor der Bisulfit-Behandlung eine Aufreinigung oder Aufkonzentration der DNA, um eine Störung der Bisulfit-Reaktion und/oder der nachfolgenden PCR durch ein zu hohes Maß an Verunreinigungen zu vermeiden. Es ist jedoch bekannt, dass beipielsweise eine PCR aus Gewebe nach Behandlung beispielsweise mit Proteinase K ohne weitere Aufreinigung erfolgen kann, und dies gilt sinngemäß auch für die Bisulfit-Behandlung und nachfolgende PCR.

Die chemische Behandlung wird bevorzugt durch Behandlung mit einem Bisulfit (=Hydrogensulft, Disulfit), wiederum bevorzugt Natriumbisulfit (weniger geeignet ist Ammoniumbisulfit) durchgeführt. Entweder erfolgt die Reaktion nach einer publizierten Variante, bevorzugt ist hier die Einbettung der DNA in Agarose, um die DNA während der Behandlung in einzelsträngigen Zustand zu halten, oder aber nach einer neuen Variante durch Behandlung in Gegenwart eines Radikalfängers und eines denaturierenden Reagenzes, bevorzugt ein Oligeothylenglykoldialkylether oder beipielsweise Dioxan. Vor der PCR Reaktion werden die Reagenzien entweder durch Waschen im Falle der Agarosemethode oder einem DNA-Aufreinigungsverfahren (Stand der Technik, Fällung oder Bindung an eine Festphase, Membran) entfernt oder aber einfach durch Verdünnung in einen Konzentrationsbereich gebracht, der die PCR nicht mehr signifikant beeinflusst.

Wesentlich für den dritten Schritt ist es nun, dass die Qualifier Positionen ausgewählt werden und eine geeignete Methode gewählt wird, die die selektive Amplifikation der zu untersuchenden DNA erlaubt. Die Auswahl der Positionen erfolgt nach der Prämisse, dass sie sich zwischen Hintergrund-DNA und zu untersuchender DNA hinsichtlich ihrer Methylierung so sehr wie möglich unterscheiden sollten. Dazu werden zunächst die Methylierungsprofile der jeweils in Frage kommenden Abschnitte eines Gens sowohl für die zu untersuchenden Tumore als auch für die Hintergrund-DNA aus gesunden Individuen bestimmt. Diejenigen Positionen, die die größten Unterschiede zwischen Tumor DNA und Hintergrund-DNA (beispielsweise im Serum) aufweisen, werden als Qualifier Positionen ausgewählt. Solche Positionen sind für eine Vielzahl von Genen bereits bekannt, beipielsweise für GSTpi, für HIC-1 und MGMT (von Wronski MA, Harris LC, Tano K, Mitra S, Bigner DD, Brent TP. (1992) Cytosine methylation and suppression of 06-methylguanine-DNA methyltransferase expression in human rhabdomyosarcoma cell lines and xenografts. Oncol Res.;4(4-5):167-74; Esteller M, Toyota M, Sanchez-Cespedes M, Capella G, Peinado MA, Watkins DN, Issa JP, Sidransky D, Baylin SB, Herman JG. (2000), Inactivation of the DNA repair gene 06-methylguanine-DNA methyltransferase by promoter hypermethylation is associated with G to A mutations in K-ras in colorectal tumorigenesis. Cancer Res. May 1;60(9):2368-71). Es gibt nun mehrere Methoden, die allesamt bevorzugt sind, mit denen man die zu untersuchende DNA unter Verwendung dieser Qualifier-Positionen bevorzugt amplifizieren kann.

Zum einen ist es möglich, wiederum eine der MSP entsprechende Reaktion durchzuführen, indem man Primer verwendet, die vollständig an die Sequenz hybridisieren, die der zu untersuchenden DNA nach Bisulfit-Behandlung entspricht, nicht aber an die analog behandelte Hintergrund-DNA. Mit anderen Worten, die Primer hybridisieren an einen DNA-Abschnitt, in dem sich eine oder mehrere Qualifier Positionen befinden, und nur wenn deren Methylierungsstatus in der ursprünglichen DNA dem der für die zu untersuchende DNA charakteristischen entspricht, kann eine Amplifikation in signifikantem Ausmass stattfinden. Dies ist eine prinzipiell einfache Variante, die hat jedoch den Nachteil, dass die Qualifier-Positionen jeweils an einem oder an beiden Enden des DNA-Fragmentes liegen müssen, d. h. die Classifier-Positionen müssen zwischen den Qualifier-Positionen liegen (oder aber, bei nur einer Qualifier Position, darf diese nicht inmitten der Clasifier-Positionen liegen). Auch wenn es erfindungsgemäß bevorzugt ist, eine solche MSP-Variante durchzuführen, ist daher davon auszugehen, dass sie nur in vergleichsweise wenigen Fällen zur Anwendung kommen kann, da die Verteilung von Qualifier und Classifier-Positionen nur in wenigen Fällen so ideal sein wird. Da prinzipiell jedoch einfach durchzuführen, ist sie hier dennoch als bevorzugt aufgeführt.

Besonders bevorzugt ist jedoch eine Variante, bei der die Primer nicht mit einer Qualifier-Position überlappen oder mit dieser hybridisieren, sondern die PCR-Amplifikation vielmehr durch mindestens ein weiteres Oligonukleotid, welches nicht als Primer fungieren kann und welches an eine Qualifier-Position bindet, beeinflusst wird.

Das heißt, dass die chemisch behandelte DNA im Prinzip, wie es Stand der Technik ist, mittels zweier Primer amplifiziert wird. Innerhalb des von den beiden Primern eingegrenzten DNA-Abschnittes befinden sich eine oder mehrere Qualifier-Positionen. Es werden nun im Gegensatz zur normalen PCR weitere Oligonukleotide zugesetzt, welche an diese Qualifier-Positionen binden, und zwar selektiv dann, wenn diese vor der Bisulfit-Behandlung entweder methyliert oder nicht methyliert vorlagen. Die zu untersuchende DNA wird demnach bevorzugt dann amplifiziert, wenn die zugesetzten Oligonukleotide weniger effektiv an ihre Qualifier Positionen binden als bei der Hintergrund-DNA. Mit anderen Worten blockieren die zugesetzten Oligonukleotide selektiv die Amplifikation der Hintergrund-DNA.

Bevorzugt enthalten diese zugesezten Oligonukleotide entweder mindestens ein CG, ein TG oder ein CA Dinukleotid. Sie müssen weiterhin die Eigenschaft haben, dass sie von der in der PCR-Reaktion eingesetzten Polymerase nicht selbst verlängert werden können. Dies geschieht bevorzugt durch den Einsatz von 3'-Desoxyoligonukleotiden oder aber an der 3'-Position anderweitig funktionalisierten Oligonukleotiden, beipielsweise 3'-O-Acetyloligonukleotiden. Weiterhin muss der Abbau dieser Oligonukleotide durch die Polymerase verhindert werden. Dies erfolgt bevorzugt entweder durch die Verwendung einer Polymerase ohne Nukleaseaktivität oder bevorzugt durch Verwendung modifizierter Oligonukleotide, die beipielsweise am 5'-Ende Thioatbrücken aufweisen und daher gegen einen Abbau resistent sind.

Eine weitere besonders bevorzugte Variante ist die Verwendung von PNA (Peptide Nucleic Acid) Oligomeren, die sinngemäß wie die Oligonukleotide in diesem Experiment eingesetzt werden. Die PNA-Oligomere werden weder von der Polymerase abgebaut und noch können sie von dieser verlängert werden, so dass diese für diese Verfahrensvariante ideal geeignet sind. Verfahren zum Design und der Synthese von DNA-Oligomeren sind Stand der Technik.

Wie oben angesprochen, können mehrere Qualifier-Positionen und auch demnach mehrere, jeweils für einen in der Hintergrund-DNA vorliegenden Methylierungsstatus spezifische Oligonukeotide in einem solchen Verfahren verwendet werden.

Nach der selektiven Amplifikation der zu untersuchenden DNA können nun bevorzugt, nach an sich bekannten Verfahren, der Methylierungsstatus mehrerer Classifier-Positionen bestimmt werden.

Es ist jedoch offensichtlich, dass auch in diesem Fall das Entstehen eines PCR Fragmentes selbst im Einzelfall von hinreichender Aussagekraft sein kann, sofern man, wie auch bei der MSP, die Situation vorliegen hat, dass die Qualifier-Position praktisch zu 100% beispielsweise in der Hintergrund-DNA unmethyliert vorliegt, jedoch in der zu untersuchenden DNA aufmethyliert vorliegt. Verwendet man nun in der PCR ein Oligonukleotid, das bevorzugt an die Sequenz bindet, welche in der Bisulfit-Behandlung aus nicht methylierter Hintergrund-DNA entsteht, so entsteht in der PCR nur dann ein Produkt, wenn wenigstens eine geringe Menge an zu untersuchender DNA überhaupt vorhanden ist. Dies kann im Einzelfall bereits hinreichend für eine Diagnose sein, und es würde sich hierbei um ein Verfahren handeln, dass ähnliche Eigenschaften aufweist wie MSP. Obgleich eine solche Durchführung nicht direkt bevorzugt ist, ist ein solches Verfahren bislang nicht bekannt und wird demzufolge ebenfalls als zugehörig zum Gegenstand dieser Erfindung betrachtet.

Bevorzugt ist es, dass in einer PCR-Reaktion mehrere Fragmente gleichzeitig erzeugt werden, d.h. dass eine Multiplex-PCR durchgeführt wird. Bei deren Design muss darauf geachtet werden, dass nicht nur die Primer, sondern auch die weiteren eingesetzten Oligonukleotide nicht züinander komplementär sein dürfen, so dass eine hochgradige Multiplexierung in diesem Fall schwieriger ist als in üblich. Jedoch hat man bei bisulfit-behandelter DNA den Vorteil, dass aufgrund des unterschiedlichen G und C-Gehaltes der beiden DNA-Stränge ein Forward-Primer niemals auch als Reverse-Primer fungieren kann, was die Multiplexierung wiederum erleichtert und diesen Nachteil im wesentlichen ausgleicht.

Im einfachsten Fall werden nun wiederum die entstandenen Fragmente nachgewiesen. Dazu kommen alle möglichen bekannten molekularbiologischen Verfahren in Frage, wie Gelelektrophorese, Sequenzierung, Flüssigchromatographie oder Hybridisierungen, ohne dass dabei die Classifier Oligonukleotide analysiert werden. Dies wäre auch zur Qualitätskontrolle der vorangehenden Verfahrensschritte denkbar. Wie oben ausgeführt, ist jedoch die nachfolgende Analyse des Methlylierungsgrades der Classifier-Positionen besonders bevorzugt.

Es gibt zahlreiche Möglichkeiten die bevorzugte Amplifikation der zu untersuchenden DNA mittels der oben beschriebenen Verfahren vorteilhaft mit Detektionstechniken für die Classifier-Oligonukleotide zu kombinieren.

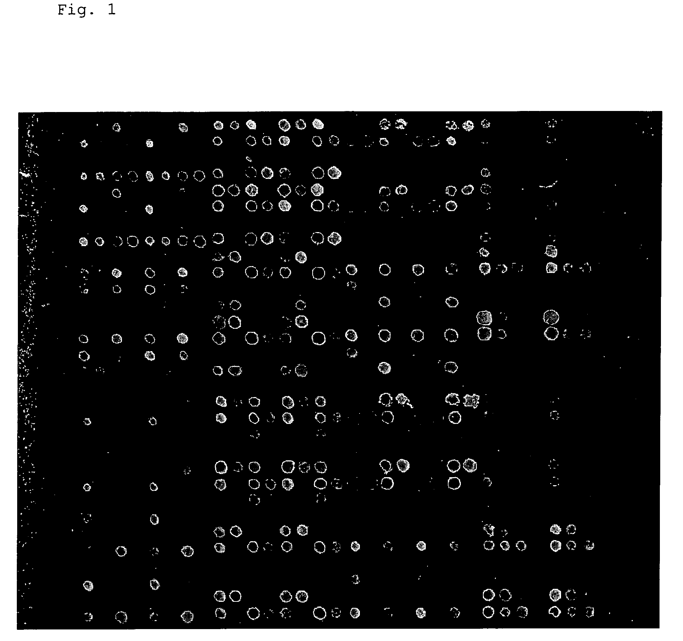

Detektionstechniken, die sich besonders eignen, sind die Hybridisierung an Oligomerarrays und beispielsweise Primer-Extension (MiniSequenzierung) Reaktionen. Die Hybridisierung an Oligomerarrays kann ohne weitere Veränderung von Protokollen gegenüber dem nächstliegenden Stand der Technik verwendet werden (Olek A, Olek S, Walter J; WO-Patent 9928498). Jedoch ist es bevorzugt, die Amplifikate an einen Array von Oligomeren zu hybridisieren, der aus Paaren von an einer Festphase immobilisierten Oligonukleotiden besteht, von denen eines jeweils stark bevorzugt an einen ein ursprünglich unmethyliertes CpG (Classifier-Position) enthaltenden DNA-Abschnitt hybridisiert und das andere wiederum stark bevorzugt an den entsprechenden Abschnitt, in dem ursprünglich ein methyliertes CpG enthalten war, jeweils vor der Bisulfit-Behandlung und Amplifikation. Besonders bevorzugt ist in diesem Fall das Amplifikat oder die Amplifikate fluoreszent oder radioaktiv oder mit ablösbaren Massentags markiert, so dass sich nach der Hybridisierung die an die beiden Oligonukleotide eines Paares gebundenen Fragmente anhand dieser Markierung nachweisen und Quantifizieren lassen. Man erhält ein Intensitätsverhältnis, aus dem man beispielsweise nach Eichung des Experimentes mit vollständig methylierter und unmethylierter DNA den Methylierungsgrad an der jeweiligen Classifier-Position bestimmen kann. Auf einem solchen Oligomer-Array (Fig. 1) lassen sich eine Vielzahl von Fragmenten und Classifier-Positionen gleichzeitig nachweisen. Es ist sinnvoll und bevorzugt, dass der Array auch Qualifier-Positionen detektierende Oligomere zur Kontrolle des Experimentes enthält, da so das Verhältnis der in die Analyse eingehenden zu untersuchenden DNA zur Hintergrund-DNA bestimmt werden kann.

Primerextensionsreaktionen können ebenfalls an auf einer Festphase immobilisierten Oligonukleotide ausgeführt werden. Obgleich nicht zwingend erforderlich, ist die Immobilisierung dieser Primer bevorzugt, da in der Regel eine Vielzahl von Classifier-Positionen aus mehreren Amplifikaten untersucht werden soll und dies auf einer Festphase, also an einem Oligomerarrays, bedeutend leichter und in einem Experiment durchführbar ist. Es ist besonders bevorzugt, dass die Primer sich unmittelbar neben einer Classifier-Position befinden und dass die Verlängerung nur um ein Nukleotid erfolgt. Es ist besonders bevorzugt, dass lediglich Didesoxythymidin und Didesoxycytidin als Nukleotide zugesetzt werden und dass diese jeweils mit einem unterschiedlichen Fluoreszenzfarbstoff markiert sind, wobei allerdings auch andere, unterscheidbare Markierungen wie Massentags denkbar und bevorzugt sind. Nach einer Bisulfit-Behandlung und Amplifikation liegen ehemalige methylierte CG als CG und nicht methylierte CG nunmehr als TG vor. Die Primerextensionsreaktion führt daher entweder zum Einbau eines Didesoxycytidins oder Didesoxythymidins. Aus dem Verhältnis der für diese beiden Terminatoren jeweils detektierten Fluoreszenzmarkierungen lässt sich auf den Methylierungsgrad der jeweiligen Position schließen. Es ist auch möglich und bevorzugt, in diesem Fall die Primerextension mit Desoxycytidin und Desoxythymidin durchzuführen, wenn man kein Guaninderivat zugibt und demzufolge bei einer TG oder CG Sequenz bereits nach einer Base die Primerextension ohnehin endet. Zudem ist es ebenfalls bevorzugt, die Analyse auf dem Gegenstrang durch Unterscheidung von CA und CG analog durchzuführen, dann entsprechend mit Didesoxy-ATP und Didesoxy-GTP oder deren Derivaten.

Eine besonders bevorzugte Variante des Verfahrens ist jedoch die gleichzeitige Detektion von Qualifier-Positionen und Classifier-Positionen in einem Experiment, was sich durch Verwendung von Taqman oder Lightcycler-Technologievarianten erzielen lässt. Dabei werden zusätzlich zu den Oligonukleotiden, die für eine bevorzugte Amplifikation der zu untersuchenden DNA sorgen, weitere fluoreszenzmarkierte Oligonukleotide zugesetzt, und die Änderung der Fluoreszenz während der PCR-Reaktion gemessen. Dabei erhält man überwiegend, weil ja hauptsächlich die zu untersuchende DNA amplifiziert wird, auch aus dieser Fluoreszenzänderung unmittelbar Information über den Methylierungsstatus verschiedener Classifier CpG Positionen. Da verschiedene Oligonukleotide bevorzugt jeweils mit unterschiedlichen Fluoreszenzfarbstoffen versehen werden, ist auch eine Unterscheidung der Fluoreszenzänderung währed der PCR getrennt für verschiedene Positionen möglich.

Diese vom Methylierungsstatus abhängige Fluoreszenzänderung kann durch zahlreiche Methoden erzielt werden, von denen hier beispielhaft zwei aufgeführt werden sollen.

Zum einen können Oligonukleotid Sonden verwendet werden, die spezifisch entweder an eine Sequenz binden, die durch chemische Behandlung aus einer an der entsprechenden Position unmethylierten DNA hervorgegangen ist, oder aber entsprechend an einer Sequenz, die durch chemische Behandlung aus einer an der entsprechenden Position methylierten DNA hervorgegangen ist. Diese Sonden sind besonders bevorzugt mit zwei Fluoreszenzfarbstoffen versehen, einem Quencherfarbstoff und einem als Marker dienenden Fluoreszenzfarbstoff. Beide sind mit der gleichen Oligonukleotidsonde verknüpft. Findet nun eine PCR-Reaktion mit der zu untersuchenden DNA als Templat statt, so wird die PCR Reaktion diesmal durch die fluoreszent markierte Oligomersonde blockiert. Da diese jedoch nicht gegen die Nukleaseaktivität der Polymerase resistent ist, findet ein Abbau der an die Templat-DNA gebundenen Sonde während der PCR-Reaktion statt, der mit der Bindungseffizienz der Sonde an das Templat korreliert, da die nicht gebundene Sonde von der Polymerase nicht abgebaut wird. Der Abbau der Sonde wird nun dadurch, dass dabei der Quencherfarbstoff und der als Marker dienende Fluoreszenzfarbstoff voneinander getrennt werden, durch eine Zunahme der Fluoreszenz des Markerfarbstoffs unmittelbar sichtbar. Im Prinzip handelt es sich hierbei um eine Variante des sogenannten Taqman Assays.

Was man demnach misst, ist das Entstehen eines PCR Produktes aus der zu untersuchenden DNA, jedoch nur dann wenn die untersuchte Classifier-Position auch in dem Methylierungszustand vorliegt, den die Sonde durch Hybridisieren an die chemisch behandelte DNA detektieren kann. Eine Gegenprobe mit einer Sonde, die entsprechend an die Classifier Position im anderen Methylierungszustand binden würde, ist daher zweckmäßig und bevorzugt.

Bevorzugt werden verschiedene Fluoreszenzfarbstoffe mit unterschiedlichen Emissionswellenlängen an mehreren Sonden zusammen mit dem Quencher eingesetzt, um eine Unterscheidbarkeit der Sonden und damit eine Multiplexierung zu erreichen.

Auch bei einem solchen Assay werden an die Qualifier Position bindende Oligonukleotide eingesetzt, die eine signifikante Amplifikation der Hintergrund-DNA verhindern. Die Amplifikation der zu untersuchenden DNA kann auch derart analysiert werden, dass man die gleiche Position auch mit einer Sonde wie oben beschrieben untersucht und die Amplifikation demnach durch eine an eine Qualifier-Position bindende Sonde nachweist. In diesem Fall ist es besonders bevorzugt, dass das nicht abbaubare Oligonukleotid selektiv an die Hintergrund-DNA bindet, während die fluoreszenzmarkierte Sonde an die zu untersuchende DNA bindet. In einer besonders bevorzugten Variante des Verfahrens weisen dabei die Sonde und das nicht abbaubare Oligonukleotid bis auf bevorzugt eine, nicht aber mehr als zwei Nukleobasen die gleiche Sequenz auf.

Besonders bevorzugt ist zudem eine Variante, bei der mehrere methylierbare Positionen als Qualifier-Positionen definiert werden und für diese Positionen je mindestens ein, an die Hintergrund-DNA bevorzugt bindendes Oligonukeotid sowie eine Sonde verwendet werden. Da die Amplifikation der Hintergrund-DNA in diesem Falle durch mehrere Oligonukleotide unterdrückt wird, eignet sich dieses Verfahren besonders in Fällen, in denen der Überschuss an Hintergrund-DNA gegenüber der zu untersuchenden DNA besonders groß ist. In vielen Fällen wird sich bei dieser Variante und dem Vorliegen mehrerer Qualifier-Positionen in einem Fragment die weitere Untersuchung von Classifier-Positionen erübrigen, da mit den heute verfügbaren Geräten auch nicht beliebig viele unterschiedliche Farbstoffe (meistens sind es 4-5) gleichzeitig detektiert werden können. Die Untersuchung weiterer Classifier-Positionen wird dann bevorzugt mit einer der anderen, oben erwähnten Detektionstechniken durchgeführt.

Bevorzugt ist es auch, dass mit einer Sonde mehrere Positionen gleichzeitig auf ihren Methylierungsgrad untersucht werden können.

Ist eine genauere Quantifizierung des Methylierungsgrades der Classifier-Positionen wünschenswert, so können bevorzugt auch zwei mit einander konkurrierende Sonden mit unterschiedlichen Farbstoffen eingesetzt werden, wobei eine wiederum im Fall einer unmethylierten Position in der zu untersuchenden DNA, die andere umgekehrt im Falle einer methylierten Position bevorzugt bindet. Aus dem Verhältnis der Fluoreszenzzunahmen für die beiden Farbstoffe lässt sich dann wiederum auf den Methylierungsgrad der untersuchten Position schließen.

Ein grundsätzlich anderes Verfahren, bei dem jedoch auch während der PCR eine Fluoreszenzänderung erfolgt, ist gegenwärtig als LightCyclertm Technologie bekannt. Dabei wird ausgenutzt, das ein Fluoreszenz Resonanz Energietransfer (FRET) zwischen zwei Farbstoffen nur erfolgen kann, wenn diese sich in unmmittelbarer Nähe, das heißt 1-5 Nukleotide voneinander entfernt befinden. Nur dann kann der zweite Farbstoff von der Emission des ersten Farbstoffes angeregt werden und dann seinerseits Licht einer anderen Wellenlänge emittieren, das dann detektiert wird.

Im vorliegenden Fall der Methylierungsanalyse erfolgt eine Hybridisierung einer fluoreszenzmarkierten Sonde an die betreffende chemisch behandelte DNA an einer Classifier-Position, und die Bindung dieser Sonde hängt wiederum davon ab, ob die zu untersuchende DNA an dieser Position methyliert oder unmethyliert vorlag. Unmittelbar benachbart zu dieser Sonde bindet eine weitere Sonde mit einem anderen Fluoreszenzfarbstoff. Diese Bindung erfolgt bevorzugt wiederum methylierungsabhängig, wenn in dem betreffenden Sequenzabschnitt eine weitere methylierbare Position vorliegt. Während der Amplifikation wird nun die DNA vermehrt, weshalb immer mehr fluoreszenzmarkierte Sonden benachbart an die betreffende Position binden, sofern diese den dafür erforderlichen Methylierungszustand aufwies, und daher sich ein zunehmender FRET gemessen wird.

Auch bei diesem Verfahren erfolgt bevorzugt eine Multiplexierung mit mehreren verschieden fluoreszenzmarkierten Sonden.

Auch hier ist es wiederum möglich und bevorzugt, dass eine Qualifier Position gemessen wird. Angenommen, dass die Hintergrund-DNA an der betreffenden Position unmethyliert vorliegt und nach chemischer Behandlung und Amplifikation ein TG Dinukleotid an dieser Position ergibt, und dass im Gegensatz die methylierte zu untersuchende DNA ein CG Dinukleotid ergibt, würde eine fluoreszenzmarkierte Sonde an die ein CG enthaltende Sequenz binden, während ein konkurrierendes Oligomer, welches nicht markiert ist, an die entsprechende TG Sequenz der Hintergund-DNA bindet. Dabei ist es wichtig, dass das unmethylierte Oligonukleotid die Amplifikation aufgrund seiner deutlich höheren Schmelztemperatur im Gegensatz zu den kürzeren Sondenolignukleotiden hemmt. Insofern sind in diesem Fall Sonden und an die chemisch behandelte Hintergrund-DNA bindende Oligomere nicht bis auf wenige Basen identisch, sondern sie sind wesentlich (um 5-15 Basen) länger. Auch ist es wiederum möglich und bevorzugt, modifizierte Oligonukeotide und/oder PNAs einzusetzen. Auch in diesem Fall sind alle Sonden und Oligonukleotide bis auf die Primer an ihrem 3'-Ende blockiert, um eine Verlängerung in der PCR zu vermeiden. Dies kann beispielsweise mit einer Phosphat-gruppe erfolgen.

Beide Verfahren unterscheiden sich im Ergebnis hauptsächlich darin, dass in einem Fall eine Abnahme, im anderen Fall eine Zunahme der Fluoreszenz gemessen wird. In beiden Fällen können sowohl Qualifier- als auch Classifier-Positionen gemessen werden.

Zusammenfassend ist ein Verfahren zum Nachweis von Cytosin-Methylierung in DNA-Proben besonders bevorzugt, bei welchem die folgenden Schritte ausgeführt werden: Zürst wird eine genomische DNA-Probe, welche zu untersuchende DNA und Hintergrund-DNA DNA umfasst, chemisch derart behandelt, dass alle nicht methylierten Cytosinbasen in Uracil umgewandelt werden, während die 5-Methylcytosinbasen unverändert bleiben, dann wird die chemisch behandelte DNA-Probe unter Verwendung von mindestens 2 Primeroligonukleotiden sowie einer Polymerase amplifiziert, wobei die zu untersuchende DNA gegenüber der Hintergrund-DNA als Templat bevorzugt wird und im nächsten Schritt werden die Amplifikate analysiert und aus dem Vorliegen eines Amplifikates und/oder aus der Analyse weiterer Positionen auf den Methylierungsstatus in der zu untersuchenden DNA geschlossen.

In einer besonders bevorzugten Verfahrensvariante wird die Proben-DNA aus Serum oder anderen Körperflüssigkeiten eines Individuums gewonnen. Ebenfalls bevorzugt ist es, dass die Proben-DNA aus Zellinien, Blut, Sputum, Stuhl, Urin, Serum, Gehirn-Rückenmarks-Flüssigkeit, in Paraffin einbettetem Gewebe, beispielsweise Gewebe von Augen, Darm, Niere, Hirn, Herz, Prostata, Lunge, Brust oder Leber, histologischen Objektträgern und allen möglichen Kombinationen hiervon gewonnen wird.

In einer besonders bevorzugten Variante des Verfahrens wird die chemische Behandlung mit einem Bisulfit (=Disulfit, Hydrogensulfit) durchgeführt. Bevorzugt ist es, die chemische Behandlung nach Einbetten der DNA in Agarose durchzuführen. Auch ist es bevorzugt, dass bei der chemischen Behandlung ein die DNA-Duplex denaturierendes Reagenz und/oder ein Radikalfänger zugegen ist.

In einer besonders bevorzugten Verfahrensvariante wird die Amplifikation im zweiten Schritt in Gegenwart mindestens eines weiteren Oligonukleotids durchgeführt, welches an ein 5'-CG-3'-Dinukleotid oder ein 5'-TG-3'-Dinukleotid oder ein 5'-CA-3'-Dinukleotid bindet, wobei das weitere Oligonukleotid bevorzugt an die Hintergrund-DNA bindet und deren Amplifikation beeinträchtigt.

Besonders bevorzugt ist es zudem, dass man die chemisch behandelte DNA-Probe im zweiten Schritt unter Verwendung von mindestens 2 Primeroligonukleotiden und einem weiteren Oligonukleotid, welches an ein 5'-CG-3'-Dinukleotid oder ein 5'-TG-3'-Dinukleotid oder ein 5'-CA-3'-Dinukleotid hybridisiert, und mindestens einem Reporteroligonukleotid, welches an ein 5'-CG-3'-Dinukleotid oder ein 5'-TG-3'-Dinukleotid oder ein 5'-CA-3'-Dinukleotid hybridisiert, sowie einer Polymerase amplifiziert; wobei das weitere Oligonukleotid bevorzugt an die Hintergrund-DNA bindet und deren Amplifikation beeinträchtigt und wobei das Reporteroligonukleotid bevorzugt an die zu untersuchende DNA bindet und deren Amplifikation anzeigt.

Es ist auch bevorzugt, dass zusätzlich zu dem Reporteroligonukleotid ein weiteres mit einem Fluoreszenzfarbstoff markiertes Oligomer verwendet wird, welches unmittelbar benachbart zu dem Reporteroligonukleotid hybridisiert und sich diese Hybridisierung mittels Fluoreszenz Resonanz Energietransfer (FRET) nachweisen lässt.

Besonders bevorzugt wird für die Analyse ein Taqman-Assay durchgeführt. Ebenso ist es bevorzugt, einen LightCycler Assay (wie oben beschrieben) durchzuführen.

Besonders bevorzugt verfügen die zusätzlich zu den Primern verwendeten Oligonukleotide nicht über eine 3'-OH Funktion. Zudem tragen die Reporteroligonukleotide besonders bevorzugt mindestens eine Fluoreszenzmarkierung.

Es ist besonders bevorzugt, dass die Reportermoleküle die Amplifikation entweder durch eine Zunahme oder eine Abnahme der Fluoreszenz anzeigen und dass die Zunahme oder Abnahme der Fluoreszenz auch direkt zur Analyse verwendet wird und aus dem Fluorenzenzsignal auf einen Methylierungszustand der zu analysierenden DNA geschlossen wird.

In einer besonders bevorzugten Verfahrensvariante erfolgt die Analyse oder die weitere Analyse mittels Hybridisierung an Oligomer-Arrays, wobei Oligomere Nukleinsäuren oder in ihren Hybridisierungseigenschaften ähnliche Moleküle wie PNAs sein können. Bevorzugt hybridisieren die Oligomere über einen 12-22 Basen langen Abschnitt an die zu analysierende DNA und umfassen ein CG, TG oder CA Dinukleotid. Mit dieser Methode wird bevorzugt der Methylierungsstatus von mehr als 20 Methylierungspositionen der zu untersuchenden DNA in einem Experiment nachgewiesen, besonders bevorzugt sind es mehr als 60 Methylierungspositionen.

Besonders bevorzugt ist auch ein Verfahren, bei dem die weitere Analyse durch Längenmessung der amplifizierten zu untersuchenden DNA erfolgt, wobei Methoden zur Längenmessung Gelelektrophorese, Kapillargelelektrophorese, Chromatographie (z.B. HPLC), Massenspektrometrie und andere geeignete Methoden umfassen.

Besonders bevorzugt ist auch ein Verfahren, bei dem die weitere Analyse durch Sequenzierung erfolgt, wobei Methoden zur Sequenzierung die Sanger-Methode, Maxam-Gilbert-Methode und andere Methoden wie Sequencing by Hybridisation (SBH) umfassen. Wiederum bevorzugt ist ein Verfahren, wobei die Sequenzierung (nach Sanger) für jede oder eine kleine Gruppe von CpG Positionen mit jeweils einem separaten Primeroligonukleotid ausgeführt wird und die Verlängerung der Primer nur eine oder einige wenige Basen ausmacht, und aus der Art der Primerverlängerung auf den Methylierungsstaus der betreffenden Positionen in der zu untersuchenden DNA geschlossen wird.

In einer besonders bevorzugten Verfahrensvariante wird aus dem Methylierungsgrad an den verschiedenen untersuchten CpG Positionen auf das Vorliegen einer Erkankung oder eines anderen medizinischen Zustandes des Patienten geschlossen.

Besonders bevorzugt sind auch die Amplifikate selbst für die Detektion mit einer nachweisbaren Markierung versehen. Bei diesen Markierungen handelt es sich bevorzugt um Fluoreszenzmarkierungen, Radionuklide oder ablösbare Massenmarkierungen, die in einem Massenspektrometer nachgewiesen werden.

Weiterhin ist ein Verfahren bevorzugt, bei dem bei der Amplifikation einer der Primer an eine Festphase gebunden ist.

Auch bevorzugt ist eine Verfahrensvariante, wobei die Amplifikate insgesamt im Massenspektrometer nachgewiesen werden und somit durch ihre Masse eindeutig charakterisiert sind.

Ein weiterer Gegenstand der vorliegenden Erfindung ist die Verwendung eines der beschriebenen Verfahren zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Zudem bevorzugt ist die Verwendung eines der beschriebenen Verfahren zur Unterscheidung von Zelltypen oder Geweben oder zur Untersuchung der Zelldifferenzierung.

Ein weiterer Gegenstand der vorliegenden Erfindung ist ein Kit, bestehend aus einem Bisulfit enthaltenen Reagenz, Primern und weiteren Oligonuleotiden ohne 3'-OH Funktion zur Herstellung der Amplifikate, sowie optional einer Anleitung zur Durchführung zumindest einer der beschriebenen Verfahrensvarianten.

Die folgenden Beispiele erläutern die Erfindung:

### Beispiel 1: Methylierungssensitive Amplifikation des MDR1 Gens unter Verwendung von PNA blocking probes (PCR clamping) im MDR-1 Gen

### a) Methylierungsspezifische PCR mit PNA Sonden (PNA blocking probes), deren Bindungsstellen nicht mit denen der Primer überlappen

Zunächst wurden PCR-Bedingungen für Bisulfit-behandelte DNA definiert, unter denen ein allelspezifischer Einfluss der PNA blocking probe auf die PCR erkennbar ist. In diesem ersten Experiment überlappen die Bindungsstellen zwischen PNA und den Primern nicht.

Zunächst wurde in einem Experiment der Einfluss einer 11 mer PNA der Sequenz AAAATGTGTT in einer PCR mit den Primern TAAGTATGTTGAAGAAAGATTATTGTAG und TAAAAACTATCCCATAATAACTCCCAAC getestet. Ein Einfluss der PNA auf die PCR Reaktion ist unter PCR-Standardbedingungen bei einer Aneealingtemperatur von 55°C nicht messbar. Das für die Amplifikation des MDR1-Fragmentes verwendete Standard-Cyclerprogramm verwendet folgende Programmschritte:

| | |
|---|---|
| Schritt 1: T=96°C | 20 min |
| Schritt 2: T=96°C | 30 s |
| Schritt 3: T=56°C | 1,15 min |
| Schritt 4: T=72°C | 2,00 min |
| Die Schritte 2 bis 4 werden als Zyklus 40 mal durchlaufen. | |
| Schritt 5: T=72°C | 15 min |
| Schritt 6: auf 4°C abkühlen und Temperatur halten | |

Demzufolge müssen die Primerlänge, die Annealingtemperatur und die PNA-Probelänge optimal angepasst werden, um eine allelspezifische Unterdrückung der Amplifikation zu erreichen.
Um den Primern und der PNA ein optimales Annealing zu ermöglichen, wurden in einer PCR Reaktion kürzere 21 mer Primer TAAGTATGTTGAAGAAAGATT und AATCCCCATAAACTTACCAAA mit einer längeren 13 mer PNA AAAGACGTGTTAT getestet. Weitere Versuche wurden mit 18, 19 und 20mer Primern durchgeführt, welche sich von den obigen Sequenzen nur dadurch unterscheiden, dass Basen am 3'-Ende weggelassen wurden. Die 21 mer Primer wurden mit einem Gradienten der Annealingtemperatur getestet. In einem Parallelansatz wurden die 13 mer PNA AAAGACGTGTTAT respektive die für eine aus einem nicht methylierten Allel hervorgegangene Sequenz passende PNA AAAGATGTGTTAT in verschiedenen Konzentrationen von 20 -100 pmol/µl zugegeben.
Ein deutlicher Einfluss auf die PCR wurde bei Annealingtemperaturen von 49,4°C und 46,7°C bei Zugabe der PNA in einer Konzentration von 70 und 100 pmol/µl beobachtet.
Der Effekt war bei der Verwendung eines 18mer Primers am deutlichsten.

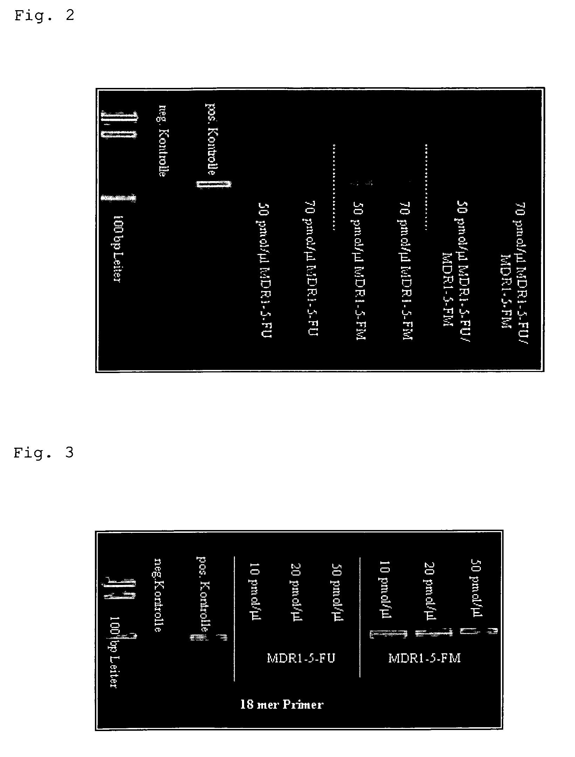

Untersucht wurde nun, inwieweit sich eine niedrigere Extensiontemperatur von 54°C auf den hemmenden Einfluss der PNAs auswirkt.
Hierfür wurden zu einem PCR-Ansatz jeweils die oben genannten 13mer PNAs bzw. beide 13mer PNAs zusammen in einer Konzentration von 50 und 70 pmol/µl gegeben.
Zu beobachten war eine deutliche Unterdrückung der PCR im Vergleich zur positiven Kontrolle ohne PNAs (Figur 2, Agarose-Gelelektrophorese, Konzentrationen der PNAs für die verwendeten 13mer PNA Sonden; Annotation: MDR1-5FM: 13 mer PNA AAAGACGTGTTAT; MDR1-5FU: 13 mer PNA AAAGATGTGTTAT). Die Versuche legen nahe, dass die in den Versuchen verwendete DNA an den betreffenden Positionen überwiegend unmethyliert vorlag, was durch Bisulfit-Sequenzierung bestätigt werden konnte. Es kann also in diesem Experiment bereits eine deutliche Allelspezifität der PNA blocking probe gezeigt werden, die zu einer bevorzugten Amplifikation der nicht methylierten Fragmente führt.

### b) Methylierungsspezifische PCR mit PNA Sonden (PNA blocking probes), deren Bindungsstellen mit denen der Primer überlappen ("primer exclusion")

In obigen Experimenten waren die Primer so gewählt, dass die PNA-Zielsequenz ungefähr in der Mitte des zu amplifizierenden Bereichs lag. Als sensitiver wurde die Anordnung beschrieben, wenn Primer- und PNA-Sequenz aneinander angrenzen bzw. sich überlappen. Bei einer sequenzspezifischen Bindung der PNA müsste bei dieser Anordnung ein Effekt der PNA schon bei geringeren PNA-Konzentrationen zu beobachten sein.

Für dieses Experiment wurde ein Primer mit der Sequenz TTATGTGAATTTTGAAAG so gewählt, dass er sich mit der PNA-Sequenz überlappt (primer exclusion). In einen Reaktionsansatz wurden beide 13mer PNAs AAAGACGTGTTAT und AAAGATGTGTTAT in 3 verschiedenen Konzentrationen zugegeben.
Eine vollständige Unterdrückung der PCR Reaktion wurde schon mit einer Konzentration von 25 pmol/µl erzielt. Im vorangegangenen Experiment war eine vollständige Unterdrückung der PCR bei Zugabe beider PNAs erst in einer Konzentration von 70 pmol/µl erkennbar.

### c) primer exclusion mit nicht methylierter DNA entsprechenden Fragmenten

Für den Nachweis einer sequenzspezifischen Bindung wurde in weiteren Experimenten die Wirkung der PNAs auf hinsichtlich des Methylierungsstatus gut charakterisierten Templaten untersucht. Die für dieses Experiment verwendete Templat DNA entsprach einer bisulfitbehandelten vollständig unmethylierten DNA.
Diese wurde als Templat in eine PCR eingesetzt. Dafür wurden die folgenden Programmschritte eingesetzt:

| | |
|---|---|
| Schritt 1: T=96°C | 20 min |
| Schritt 2: T=96°C | 30 s |
| Schritt 3: T=49°C | 1,15 min |
| Schritt 4: T=54°C | 2,00 min |
| Die Schritte 2 bis 4 werden als Zyklus 36 mal durchlaufen. | |
| Schritt 5: T=72°C | 15 min |
| Schritt 6: auf 4°C abkühlen und Temperatur halten | |

Zum Reaktionsansatz wurden die 13 mer wie oben beschrieben in 3 verschiedenen Konzentrationen zugegeben. In diesem Fall des unmethylierten Templates würde man erwarten, dass die für dieses Templat passende PNA MDR1-5-FU (3) einen deutlich stärkeren Einfluss als MDR1-5-FM (3) hat. In Figur 3 (Annotation siehe Figur 2) ist dargstellt, dass dies auch bei vergleichsweise niedrigen PNA Konzentrationen der Fall ist.

Diese Ergebnisse zeigen, dass die Unterdrückung der PCR Reaktion durch spezifisch bindende PNAs methylierungsspezifische Amplifikationen ermöglicht. In Kontrollexperimenten mit nicht zu MDR-1 komplementären PNAs konnte gezeigt werden, dass diese keinen nennenswerten Einfluss auf die PCR bei den in Frage kommenden Konzentrationen ausüben (nicht gezeigt).

### Beispiel 2: Methylierungssensitive Amplifikation eines Fragments des GSTpi Gens

Bestimmte CpG-Positionen des GSTPi-Genes wurden als Tumormarker für Prostatakrebs identifiziert.
Bei dem für die Experimente ausgewählten Primerpaar GGAAAGAGGGAAAGGTTTT und TACTAAAAACTCTAAACCCCAT ist ein Primer so lokalisiert, das er genau an die PNA-Sequenz CCCCGAAAACGCG (bzw, CCCTGAAAATGTG) angrenzt. Die PNA-Sequenz umfasst im Unterschied zu den für das MDR1 Fragment verwendeten PNAs nun drei relevante CpG Positionen. Die zu untersuchenden relevanten CpGs des GSTPi Fragmentes liegen in "normaler", d.h. nicht von Tumorpatienten stammender DNA nicht methyliert vor. Die zum Reaktionsansatz gegebene PNA "GSTP-down" der Sequenz CCCTGAAAATGTG sollte daher einen erkennbaren Einfluss auf die PCR-Reaktion haben, jedoch nicht die entsprechende PNA "GSTP-up" CCCCGAAAACGCG.
Zum Versuchsansatz wurde die PNA "GSTP-down" in drei verschiedenen Konzentrationen zugegeben. Getestet wurde ein Gradient der Annealingtemperatur.

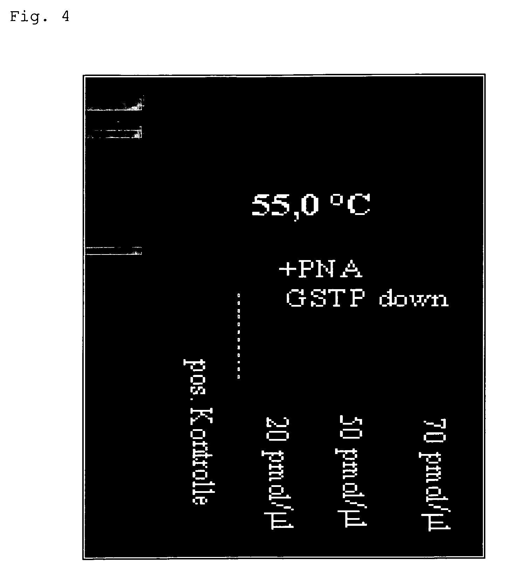

Die Ergebnisse des Experimentes sind in Figur 4 daargestellt. Die stärkste Unterdrückung der PCR durch Zugabe der PNA (GSTP-down) ist bei einer Annealingtemperatur von 55°C feststellbar. Im Vergleich mit der positiven Kontrolle (ohne Zugabe von PNA) ist erkennbar, dass die PCR Reaktion schon durch die Zugabe der PNA in einer Konzentration von 20 mol/µl signifikant unterdrückt werden konnte.

Nachfolgend wurde, um eine sequenzspezifische (und damit letztlich methylierungsensitive) Bindung nachzuweisen, der Einfluss der PNAs "GSTP-up" und "GSTP-down" auf die Amplifikationen einer in der ursprünglichen Probe unmethylierten DNA und einer aus einem Prostatatumorgewebe stammenden methylierten DNA als Templat gegenübergestellt.
Die unmethylierte DNA und die Prostata DNA wurden als Templat in eine PCR eingesetzt. Zum Reaktionsansatz wurden die PNAs "GSTP-up" bzw. "GSTP-down" in drei verschiedenen Konzentrationen zugegeben.

Die Zugabe der PNA "GSTP-down" hat auf dem downmethyliertem Templat einen sichtbaren Einfluss: die PCR ist bei Zugabe der PNA in einer Konzentration von 70 pmol/µl vollständig unterdrückt. Bei Zugabe der PNA "GSTP-up" ist dagegen nur ein schwacher, hemmender Einfluss der PNA auf die PCR erkennbar.

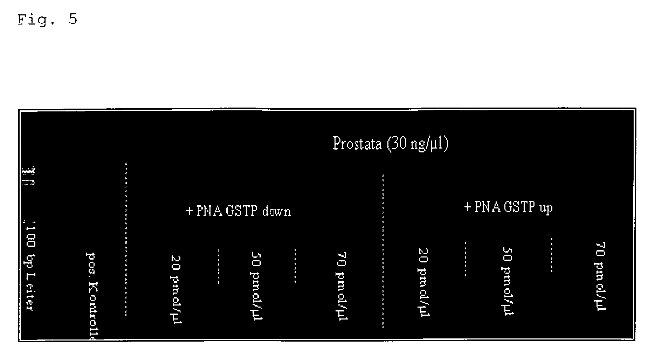

Die Zugabe der PNA "GSTP-up" zur Test-DNA aus Prostatagewebe hat einen erheblich hemmenden Einfluss auf die PCR (Figur 5). Die Zugabe der PNA in einer Konzentration von 20 pmol/µl unterdrückt die PCR schon deutlich. Eine vollständige Unterdrückung der PCR ist bei einer Zugabe der PNA in einer Konzentration von 50 pmol/µl feststellbar. Im Gegensatz dazu hat die Zugabe der PNA "GSTP-down" zur Prostata DNA einen deutlich geringer Einfluss. Auch eine Zugabe der PNA in einer Konzentration von 70 pmol/µl unterdrückt die PCR nicht vollständig.

Die Experimente zeigen, dass es möglich ist, mittels blockierender Oligomersonden selektiv die Amplifikation von an definierten Positionen methylierten oder unmethylierten Allelen zu unterdrücken. Im Sinne dieser Erfindung würden die betreffenden Positonen als Qualifier-Positionen dienen, d.h. man würde selektiv die Amplifikation unerwünscht methylierter Template der Hintergrund DNA unterdrücken.

### Beispiel 3: Verschiedene Möglichkeiten des Einsatzes von methylierungsspezifisch die PCR unterdrückenden Sonden am Beispiel des GSTPi Gens

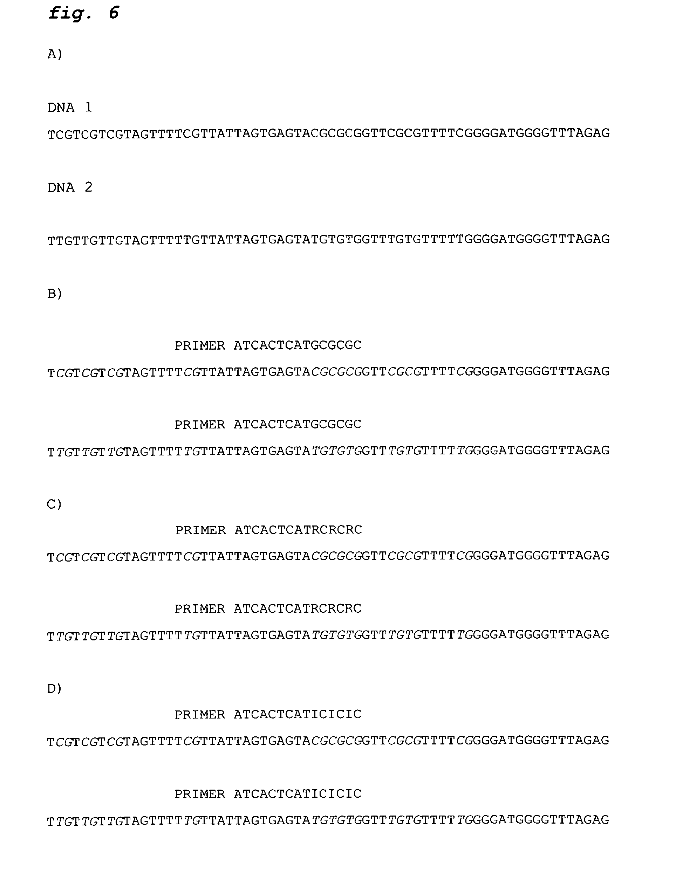

In Figur 6 sind mehrere Möglichkeiten dargestellt, wie bei einer gegebenen Templatsequenz Primer im Sinne einer methylierungsensitiven Amplifikation anzuordnen sind. Zur Erläuterung zeigt Figur 6a die nach der Bisulfitbehandlung vorliegenden Template, DNA 1 entsprechend einer ursprünglich methylierten DNA-Probe, DNA 2 entsprechend einer ursprünglich unmethylierten DNA-Probe.
Figur 6b zeigt die Anordnung eines der Primer im Sinne einer Allelspezifischen PCR bzw. einer Methylierungsspezifischen PCR (MSP). In diesem Fall würde nur die Amplifikation der methylierten DNA 1 unter Verwendung des gezeigten Primes stattfinden können.

Figuren 6c und 6d zeigen, wie entsprechend nicht methylierungsspezifische Primer eingesetzt werden können, entweder durch Verwendung degenerierter Positionen (6c) oder aber universeller Basen (hier Inosin) in Figur 6d.

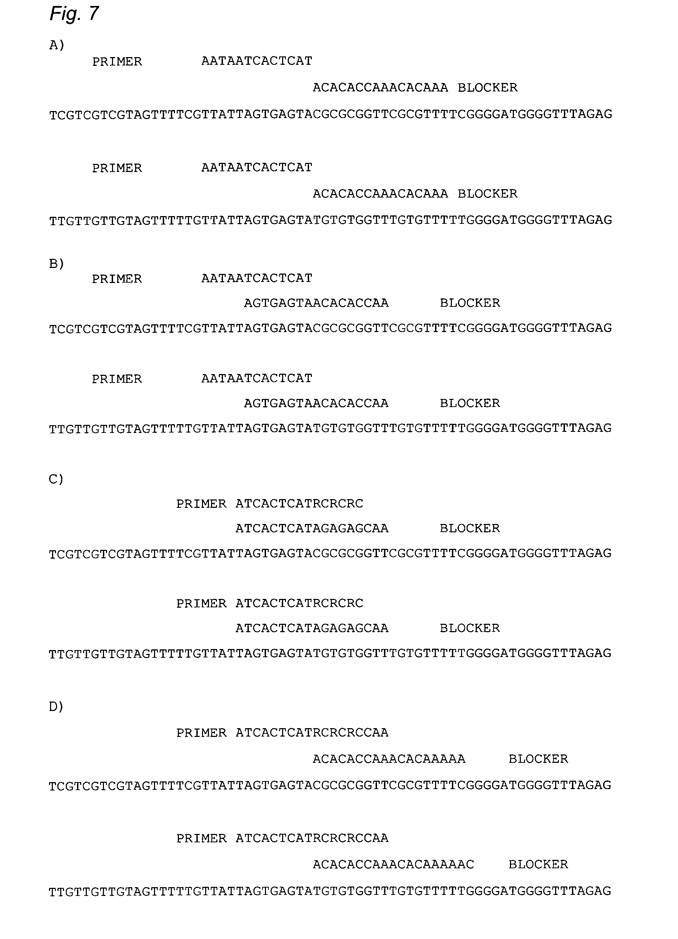

In Figur 7 sind mehrere Möglichkeiten dargestellt, wie bei einer gegebenen Templatsequenz Primer und Sonden ("Blocker") im Sinne einer methylierungsensitiven Amplifikation anzuordnen sind. In diesen Beispielen sind die verwendeten Primer nicht selbst methylierungsspezifisch, sonden die Methylierungsspezifität wird allein durch die Sonden ("Blocker") erreicht. Für DNA 1 und DNA 2 werden jeweils spezifische Sonden als Blocker eingesetzt.

In Figur 7a überlappen Primer und Sonde nicht, sondern die Sonde schliesst sich unmittelbar an das 3'-Ende des Primers an. Die dargstellte Sonde ist ein am 3'-Ende modifiziertes Oligonukleotid, das in der Amplifikation nicht selbst verlängert werden kann. Analog können auch PNAs verwendet werden, die in diesem Beispiel jedoch entsprechend ihrer Schmelztemperatur kürzer sein müssten. In Figur 7b wird der gleiche Primer eingesetzt, jedoch überlappt hier die DNA-Sonde mit dem Primer (Primer Exclusion).

In Figur 7c und 7d überlappen ein mit degenerierten Positionen versehener Pimer und die methylierungsspezifische Sonde. Analog sind auch universelle Basen in den Primern einsetzbar.

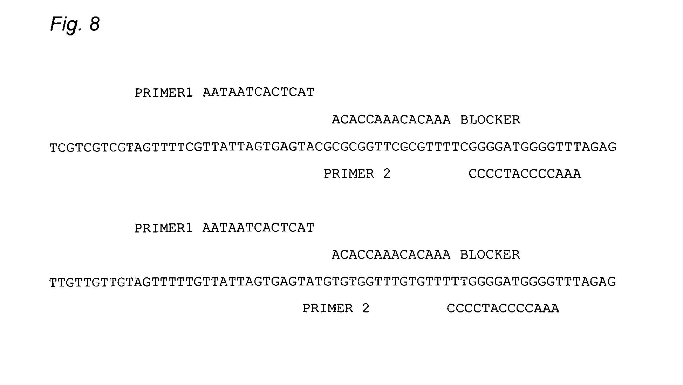

In Figur 8 ist analog ein Beispiel mit Forward und Reverse-Primer dargestellt, in dem eine Sonde verwendet wird, die mit keinem der Primer überlappt.

Diese Beispiele sollen die zahlreichen Möglichkeiten veranschaulichen, wie Oligomersonden zur methylierungsspezifischen Amplifikation eingesetzt werden können, um Hintergrund DNA gegenüber der zu analysierenden DNA zu unterdrücken. Der Umfang der Erfindung soll sich jedoch nicht auf die hier beipielhaft angeführten Ausführungen beschränken.

### Beispiel 4: Herstellung von nicht- und aufmethylierter DNA und Bisulphit-Behandlung

Für die Herstellung aufmethylierter DNA wurden humane genomische DNA mit S-Adenosylmethion und der CpG Methylase (SssI, New England Biolabs) nach Angaben des Herstellers behandelt. Für die Herstellung nicht-methylierter DNA wurde das Genfragment ELK-1 mit den Primern GCTCTATGGTCTTGTCTAACCGTA (SEQ-ID: 1) und AGGTGGTGGTGGCGGTGG (SEQ-ID: 2) ausgehend von humaner genomischer DNA, mittels PCR amplifiziert. Die so hergestellte nicht- und aufmethylierte DNA, wie auch humane genomische DNA, wurde unter Verwendung von Bisulphit (Hydrogensulfit, Disulfit) derart behandelt, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben. Wird für die Reaktion Bisulfit im Konzentrationsbereich zwischen 0.1 Mol und 6 Mol verwendet, so findet an den nicht methylierten Cytosinbasen eine Addition statt. Zudem müssen ein denaturierendes Reagenz oder Lösungsmittel sowie ein Radikalfänger zugegen sein. Eine anschließende alkalische Hydrolyse führt dann zur Umwandlung von nicht methylierten Cytosin-Nukleobasen in Uracil. Diese umgewandelte DNA dient dazu, methylierte Cytosine nachzuweisen.

### Beispiel 5: Herstellung der Cy5-markierten Gensonden

Ausgehend von den Bisulphit behandelten DNA Proben wird je ein definiertes Fragment der Länge 595 bp aus der Promotorregion des ELK-1 Gens amplifiziert. Die Amplifikation wird mit den Primeroligonukleotiden ATGGTTTTGTTTAATYGTAGAGTTGTTT (SEQ-ID: 3) und TAAACCCRAAAAAAAAAAACCCAATAT (SEQ-ID: 4) durchgeführt. Durch Verwenden von Primeroligonukleotiden, die mit dem Fluoreszenfarbstoff Cy5 markiert sind, wird das Fragment direkt bei der PCR markiert. Als Matrix DNA wird Bisulphit (Hydrogensulfit, Disulfit) behandelte (1) nichtmethylierte, (2) aufmethylierte und (3) humane genomische DNA verwendet. Anschließend werden diese drei unterschiedlichen DNA-Fragmente in getrennten Hybridisierungen auf ihren Methylierungsgrad an einer spezifischen CpG Position untersucht.

### Beispiel 6: Durchführung der Hybridisierung und Auswertung eines hybridisierten DNA-"Chip"

Die in Beispiel 5 hergestellten Gensonden werden auf einem DNA Chip hybridisiert. Auf dem Chip sind zuvor Oligonukleotide immobilisiert worden. Die Oligonukleotidesequenzen leiten sich von dem in Beispiel 2 genannten amplifizierten Fragment des Gens ELK-1 ab, und repräsentierten die CG Dinukleotide, die unmittelbare Umgebung einschließend. Die Länge der Oligonukleotide beträgt 14-22 Nukleotide, die Position des CG Dinukleotides innerhalb der Oligonukleotide ist variabel. Nach der Hybridisierung wird der DNA Chip gescannt (siehe Fig. 1) und die Hybridisierungssignale numerisch ausgewertet (Daten nicht gezeigt). Das Ergebnis der Hybridisierung für die Oligonukleotide CTACTCAACGAAAACAAA (SEQ-ID: 5) und CTACTCAACAAAAACAAA (SEQ-ID: 6) wird in Fig. 1 gezeigt. Hierbei hybridisiert CTACTCAACGAAAACAAA (SEQ-ID: 5) bevorzugt, wenn das zu Cytosin des ELK-1 Fragments, welches sich an Position 103 des Amplifikates befindet, methyliert ist, CTACTCAACAAAAACAAA (SEQ-ID: 6) wenn dieses Cytosin nicht-methyliert ist.

In Figur 1 ist ein DNA Chip nach Hybridisierung mit dem Promotor Fragment dargestellt. Dargestellt ist das Falschfarben-Bild wie es nach dem Scannen erzeugt wird. Entgegen der hier dargestellten schwarz-weiß Abbildung wird von dem Scanner ein farbiges Bild erzeugt. Die Intensität der verschiedenen Farben repräsentiert den Grad der Hybridisierung, wobei der Hybridisierungsgrad von rot (in Figur 1 als helle Spots zu erkennen) nach blau (in Figur 1 als dunkle Spots zu erkennen) abnimmt.

### Beispiel 7: Herstellung der Templat-DNA und Etablierung der GSTp1 PCR

Als Templat-DNA diente humane DNA aus peripherem Blut (Promega, Madison USA), unbehandelt und in vitro enzymatisch methyliert, die einer Bisulfit-Behandlung unterzogen wurde. Für die Methylierung aller CG-Dinukleotid wurden 6 µg DNA in einem Reaktionsvolumen von 150 µl mit SssI (New England Biolabs,Frankfurt/Main) nach Herstellerangaben umgesetzt. Die Bisulfit-Behandlung erfolgte nach einem publiziertem Verfahren (Olek A, Oswald J, Walter J. A modified and improved method for bisulphate based cytosine methylation analysis. Nucleic Acids Res. 1996 DEC 15;24(24):5064-6).

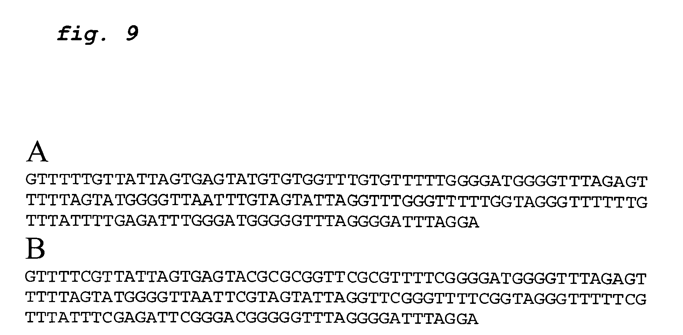

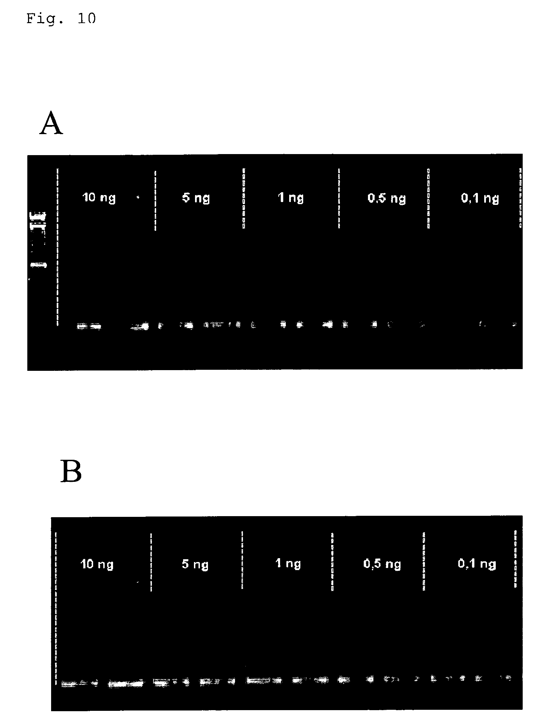

Ein 153 bp GSTp1 Fragment (Position 1242-1393 in Sequenz Acc-Nr M24485.1) wurde mit den Bisulfit-DNA spezifischen Primer 2cf GTTTT(CT)GTTATTAGTGAGT und 2cr TCCTAAATCCCCTAAACC in einem Reaktionsvolumen von 25 µl (1x Reaktionspuffer, Qiagen; 1 U HotstarTaq, Qiagen; dNTPs jedes 200 µM, jeder Primer 500 nM , 0,05-10 ng Bisulfit-behandelte Templat-DNA) unter folgenden PCR-Bedingungen (95 °C - 15 min; 46 Zyklen: 96 °C - 0:45 min, 52 °C - 0:45 min, 72 °C - 0:20 min; 72 °C - 10 min) amplifiziert (siehe Figur 9 und 10). Durch Sequenzierung der GSTp1 Fragmente konnte gezeigt werden, dass humane DNA aus peripherem Blut für dieses Fragment keine methylierten CG-Dinukleotide hat, wogegen in der SssIbehandelten DNA alle CG-Dinukleotide in der methylierten Form vorliegen (siehe Figur 9). Die Sequenzierung des GSTp1-Fragment bestätigtet Ergebnisse andere (siehe z.B: WO 9955905), dass im GSTp1 Gen, im Gegensatz zur publizierten Sequenz (Genbank Acc-Nr. M24485.1), eine zusätzliche G - Nukleotid vorhanden ist (zwischen den Positionen 1273 und 1274 in Genbank Acc-Nr. M24485.1; Position 33 im GSTp1 PCR-Fragment, siehe Figur 9). Bezüglich der PCR-Effizienz besteht kein Unterschied zwischen der CpGmethylierten und CpG-unmethylierter Templat-DNA (siehe Figur 10).

### Beispiel 8: Selektive Amplifikation methylierter GSTp1-Fragmente.

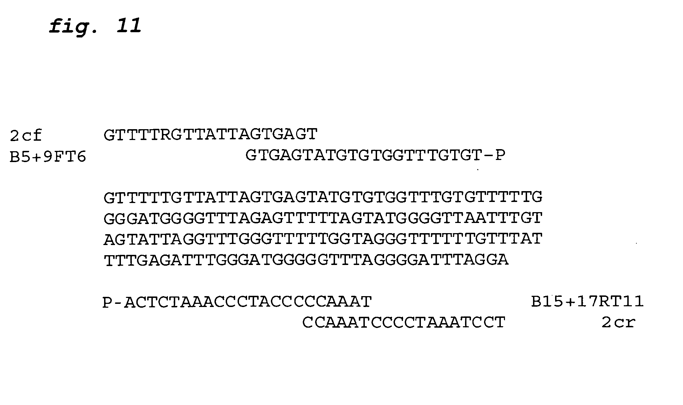

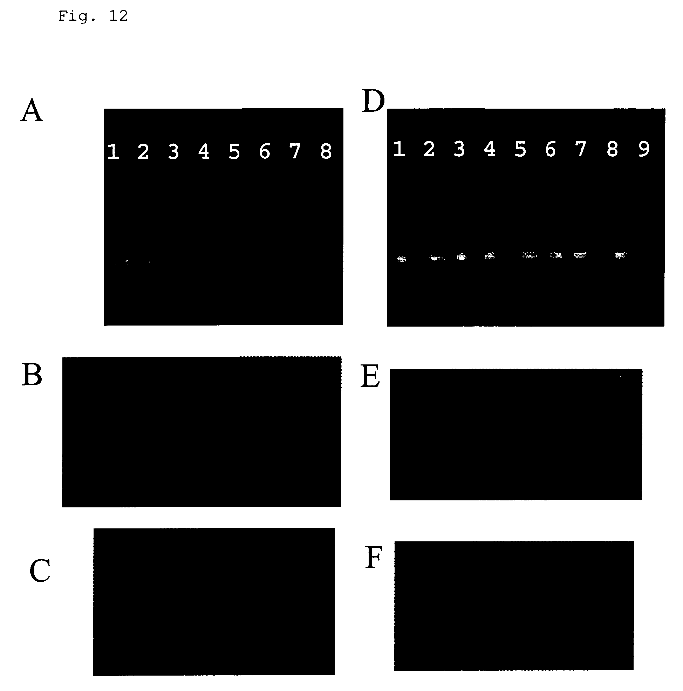

In Figur 11 ist schematisch das Experimentdesign für die selektive Amplifikation methylierter GSTp1-Fragmente dargestellt. Die Amplifikation des GSTp1-Fragments mit den Primer 2cf GTTTT(CT)GTTATTAGTGAGT und 2cr TCCTAAATCCCCTAAACC auf unmethylierter Templat-DNA wird durch zwei Blocker-Oligonukleotide (B5+9FT6, GTGAGTATGTGTGGTTTGTGT-P; B15+17RT11, TAAACCCCCATCCCAAATCTCA-P, siehe Figur 11) deren Sequenz der unmethylierten, Bisulfit-behandelter DNA entsprechen, verhindert. Diese Oligonukleotide sind am 3'-Ende durch eine Phosphat-Gruppe modifiziert, um deren Extension während der PCR zu verhindern. Die PCR wurde in einem Reaktionsvolumen von 25 µl mit folgendem Cyclerprogramm (95 °C - 15 min; 46 Zyklen: 96 °C - 0:45 min, 52 °C - 0:45 min, 72 °C - 0:20 min; 72 °C - 10 min) durchgeführt. Der PCR-Ansatz war wie folgt zusammengesetzt: lx Reaktionspuffer (Qiagen, Hilden); 2 U HotstarTaq (Qiagen, Hilden); dNTPs jedes 200 µM, jeder Primer 500 nM, jeder Blocker 10 µM (B5+9FT6, GTGAGTATGTGTGGTTTGTGT-P und B15+17RT11, TAAACCCCCATCCCAAATCTCA-P), 20 ng-20 pg Bisulfit-behandelte Templat-DNA. Unter diesen PCR-Bedingungen war es möglich die Amplifikation des GSTp1-Fragments auf 25 µg unmethylierter Templat-DNA vollständig zu unterdrücken (siehe Figur 12 A Spur 8). Wurde die PCR ohne Blocker-Oligonukleotide durchgeführt wurde das GSTP1-Fragment amplifiziert (siehe Figur 12, D Spur 8). Hingegen konnte das GSTp1-PCR-Produkt unter den gleichen PCR-Bedingungen, mit und ohne Blocker-Oligonukleotide, auf 100 pg methylierter Templat-DNA detektiert werden (siehe Figur 12, C Spur 7, F Spur 7). Die absolute Sensitivität der PCR liegt somit bei mindestens 100 pg methylierter Templat-DNA.

Um die relative Sensitivität der PCR zu untersuchen, wurden von unmethylierter und methylierter Templat-DNA Mischungen hergestellt, in denen das Verhältnis unmethylierter zu methylierter DNA 1:1 bis 1:1000 betrug. Für die Herstellung dieser DNA-Mischungen wurde humane DNA aus peripherem Blut (Promega Madison; USA), mit SssI behandelter DNA (siehe Beispiel 7), entsprechend der zu erzielenden Verhältnisse gemischt und dann einer Bisulfit-Behandlung unterzogen. Die Ergebnisse der PCR auf diesen Templat-DNA-Mischungen (25 µg Gesamt-DNA), durchgeführt mit und ohne Blocker-Oligonukleotide, sind in Figur 12 A,B bzw. Figur 12 D,E dargestellt. Sie zeigen, dass eine Kopie des methylierten GSTp1 Gen in einem Hintergrund von 200 Kopien des unmethylierten GSTP1 Gens reproduzierbar detektiert werden kann (siehe Figur 12 A Spur 6, B Spur 6). Eine relative Sensitivität von 1:1000 scheint durch weitere Optimierung der PCR-Bedingung erreichbar (siehe Figur 12 B Spur 7).

Die Sequenzanalyse der PCR-Produkte, amplifiziert aus der DNA-Mischung (1:200, Verhältnis von unmethylierter zu methylierter DNA), mit (siehe Figur 12 A Spur 6) und ohne B (siehe Figur 12 D Spur 6)Blocker zeigten das erwartete Resultat. Das PCR-Produkt erzeugt in der PCR ohne Blocker, entsprach einem unmethylierten GSTp1-Gen, wogegen das GSTp1-Genfragment, erzeugt in der PCR mit Blocker-Oligonukleotiden einen methylierten epigenetischen Status hatte.

Andere 3'-Modifikation, wie ddNTP oder zusätzliche Nukleotide, die nicht der entsprechenden GSTp1-Nukleotidsequenz entsprechen, wurden auch erfolgreich getestet.

### Beispiel 9: Selektive Amplifikation methylierter GSTp1-Fragmente auf einem LightCycler.

Der LightCycler (Roche) ist ein Gerät zur Durchführung von PCR und gleichzeitiger Detektion und Analyse der PCR-Produkte. Die Handhabung des Gerätes erfolgte nach Herstellerangaben. Die quantitativ und qualitative Analyse der PCR erfolgte mit der LightCycler Software Version 3.5.

Die selektive Amplifikation methylierter GSTp1-Genfragmente wurde in 10 µl Reaktionvolumen (1x Reaktionspuffer (Qiagen, Hilden); 5 U HotstarTaq (Qiagen, Hilden); jedes dNTPs 250 µM, jeder Primer 625 nM (2cf, GTTTT(CT)GTTATTAGTGAGT; 2cr, TCCTAAATCCCCTAAACC, Blocker 4 µM (B5+9FT16, GTGAGTATGTGTGGTTTGTGTT-P), 0,25 µg/µl BSA (Sigma, München), 250 nM Anker-Oligonukleotide (GSTp1-Fluo, TTTAGAGTTTTTAGTATGGGGTTAATT-Fluorescein; TibMolBiol, Berlin),250nM Hybridisierungssonde (GSTp1-Red 705, Red705-GTATTAGGTTTGGGTTTTTGGT-P; TibMolBiol, Berlin) und/oder GSTp1-Red 650, Red650-TAGTATTAGGTTCGGGTTTTCGG-P, TibMolBiol, Berlin), 20 ng-20 pg Templat-DNA) mit folgendem Cyclerprogramm durchgeführt: 95 °C - 15 min; 46 Zyklen: Denaturierung 96 °C - 4 s, Annealing 52 °C - 30 s, Extension 72 °C - 20 s. Die Detektion erfolgte bei jedem Amplifikationzyklus durch Gen- und Methylierungsspezifische LightCycler-Detektionssonden im Annealingschritt nach 10 s. Die Detektion des GSTp1-PCR-Fragments erfolgte, wenn sowohl die methylierungsunspezifische Anker-Sonde,GSTp1-Fluo sowie eine der methylierungsspezfischen Sonden GSTp1-Red 705 oder GSTp1-Red 650 mit dem PCR-Fragment hybridisieren.

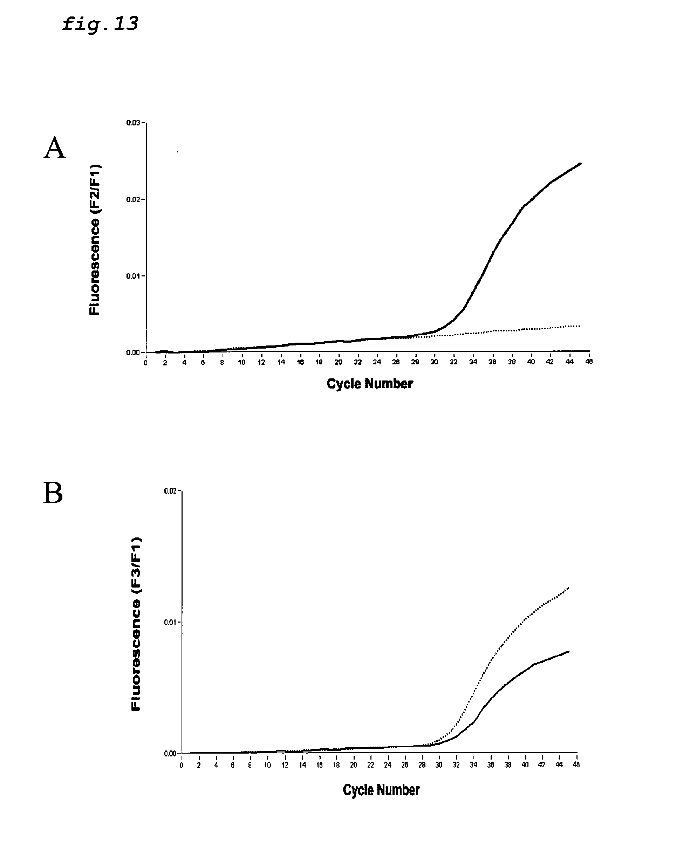

Zur Überprüfung der Methylierungsspezifität der Detektionssonden wurden je 15ng Bisulfit-behandelte methylierte und unmethylierte Templat-DNA im LightCycler amplifiziert. Zur Detektion enthielt die PCR die Ankersonde GSTp1-Fluo und eine äquimolare Mischung der Hybridisierungssonde GSTp1-Red 705 und GSTp1-Red 650. Fluoreszenz der Sonde, für das methylierte GSTp1-Gen. GSTp1-Red 650 wurde im F2/F1-Detektionskanal des LightCycler gemessen, wogegen die Sonde für das unmethylierte GSTp1-Gen, GSTp1-Red 705, im Kanal F3/F1 detektiert wird (siehe Figur 13). Die Experimente zeigten, dass GSTp1-Red 650 spezifisch das methylierte GSTp1-Gen detektiert, die unmethylierte Version erzeugte kein Fluoreszenzsignal (siehe Figur 13 A). Die Sonde GSTp1-Red 705 detektiert, hingegen das unmethylierte, und das methylierte GSTp1-Gen, das letztere mit signifikant verringerter Effizienz (siehe Figur 13 B).

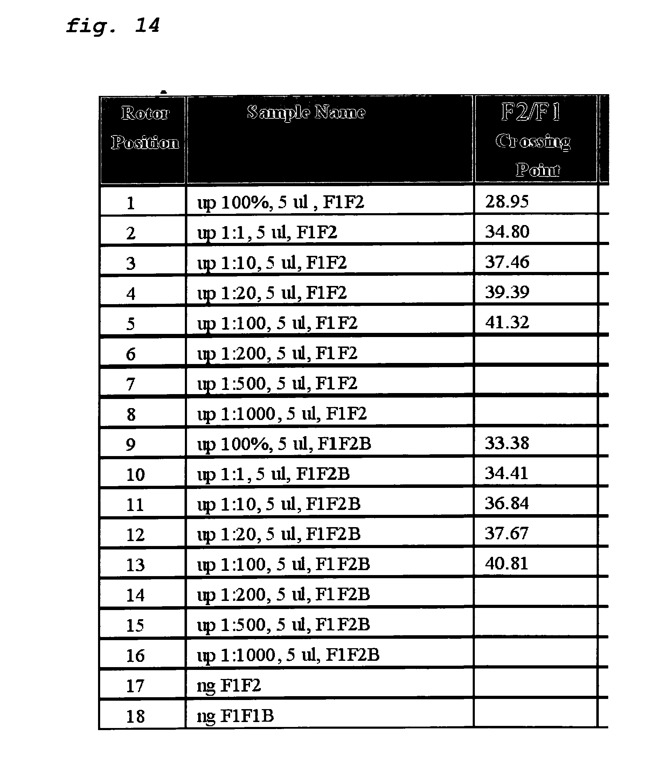

Die absolute und relative Sensitivität der Amplifikation des methylierter GSTp1-Fragments wurde in Analogie zum Beispiel 8 untersucht. Zur Bestimmung der absoluten Sensitivität wurden die GSTp1-PCR auf unterschiedlichen Mengen methylierte Bisulfit-behandelte Templat DNA im LightCycler mit und ohne Blocker-Oligonukleotid durchgeführt. Für die Detektion wurde neben der "Anker"-Sonde die Hybridisierungssonde, GSTp1-Red 650, verwendet. Die Ergebnisse sind in Figur 14 zusammen gefasst. Die Berechnung der "crossing points" erfolgte durch die LightCycler Software Version 3.5 und gibt die Anzahl der PCR-Zyklen an, bei der das GSTP1 PCR-Produkt das erste Mal, mit einem höheren Signal als die Negativkontrolle, detektiert werden konnte. Dies bedeutet, je niedriger der "crossing point"-Wert, um so effizienter erfolgte die Amplifikation des GSTp1-Fragments. Keine Angabe des "crossing point" bedeutet, dass kein PCR-Produkt detektiert werden konnte. Im dargestellten Experiment konnte das GSTp1, auch in Anwesenheit des Blockers, mit 75 pg methylierter Bisulfit-behandelter Templat-DNA amplifiziert werden (siehe Figur 14).

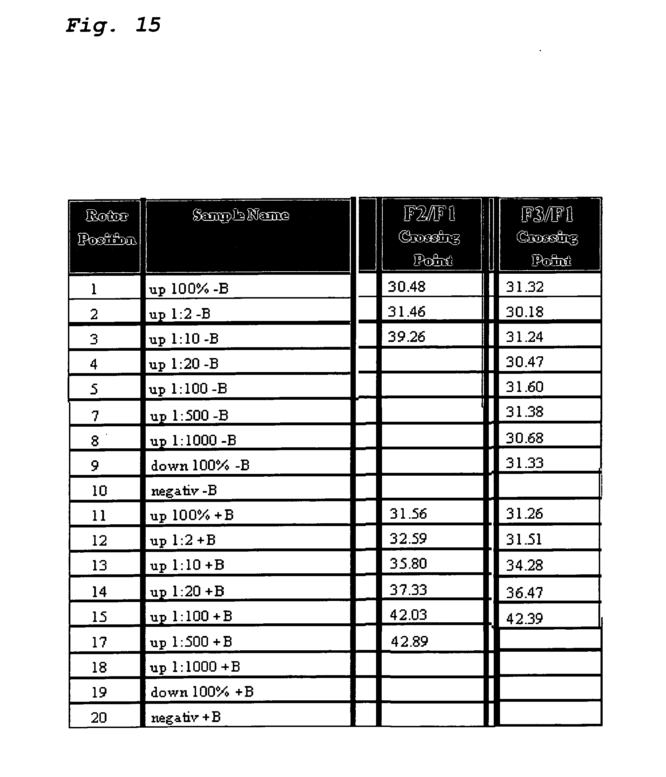

Zur Bestimmung der relativen Sensitivität wurden PCR von GSTp1 mit 20 ng der Templat-DNA-Mischungen (siehe Beispiel 8) mit und ohne Blocker-Oligonukleotide durchgeführt (Figur 15). Zur Detektion enthielt die PCR die "Anker-Sonde GSTp1-Fluo und eine äquimolare Mischung der Hybridisierungssonde GSTp1-Red 705 und GSTp1-Red 650. Fluoreszenz der Sonde, für das methylierte GSTp1-Gen. GSTp1-Red 650 wurde im F2/F1-Detektionskanal des LightCycler gemessen, wogegen die Sonde für das unmethylierte GSTp1-Gen, GSTp1-Red 705, im Kanal F3/F1 detektiert wird.

Die ermittelten "crossing point" zeigten, dass in einer PCR mit Blocker-Oligonukleotid eine Kopie des methylierten GSTp1 Gen, in einem Hintergrund von 500 Kopien des unmethylierten GSTP1 Gens reproduzierbar detektiert werden kann (siehe Figur 15, "rotor position 17, F2/F1 Spalte). Dies entspricht einer absoluten Sensitivität von 40 pg methylierter Templat-DNA. Ohne Blocker-Oligonukleotide konnte lediglich eine relative Sensitivität von 1:10 erreicht werden (siehe Figur 15, "rotor position 3, F2/F1 Spalte). Unter den gleichen Bedingungen werden die Amplifikation des GSTp1-Gens von 15 ng unmethylierter Bisulfit-behandelter Templat-DNA komplett unterdrückt (siehe Figur 15 , "rotor position 19 u. 9, F3/F1 Spalte).

### Beispiel 10: Selektive Amplifikation methylierter GSTp1-Fragmente auf einem TaqMan.

Der TagMan (Applied Biosystems,Weiterstadt) ist ein weiteres Gerät zur Durchführung von PCR und gleichzeitiger Detektion und Analyse der PCR-Produkte. Die Handhabung des Gerätes erfolgte nach Herstellerangaben. Die quantitative und qualitative Analyse der PCR erfolgte mit der TaqMan Software.

Die selektive Amplifikation methylierter GSTp1-Genfragmente wurde in 20 µl Reaktionvolumen (1x Reaktionspuffer (Applied Biosystems); 2 U Amplitaq Gold (Applied Biosystems); 3,5 mM MgCl2, jedes dNTPs 400 µM, jeder Primer 500 nM (2cft, GTTTT(CT)GTTATTAGTGAGTA ; 2cr, TCCTAAATCCCCTAAACC, Blocker1 7,5 µM (B5+9FT6, GTGAGTATGTGTGGTTTGTGT-P), Blocker2 7,5 µM (B15+17RT19, TAAACCCCCATCCCAAATCTC-P), 450nM TaqMan-Sonde (Taq1, Black hole-TAATTCGTAGTATTAGGTTCGGGTTTTCGGTAGGG-FAM; Biosearch Technologies), 10 ng Templat-DNA) mit folgendem Cyclerprogramm durchgeführt: 95 °C - 10 min; 3 Zyklen: Denaturierung 96 °C - 15 s, Annealing 60 °C - 60 s; 3 Zyklen: Denaturierung 96 °C - 15 s, Annealing 58 °C - 30 s, Extension 60 °C - 30 s; 3 Zyklen: Denaturierung 96 °C - 15 s, Annealing 55 °C - 30 s, Extension 60 °C - 30 s; 40 Zyklen: Denaturierung 96 °C - 15 s, Annealing 52 °C - 30 s, Extension 60 °C - 40 s). Die Detektion erfolgte bei jedem Amplifikationzyklus durch die Gen-spezifische TaqMan-Sonden nach dem Extensionschritt.

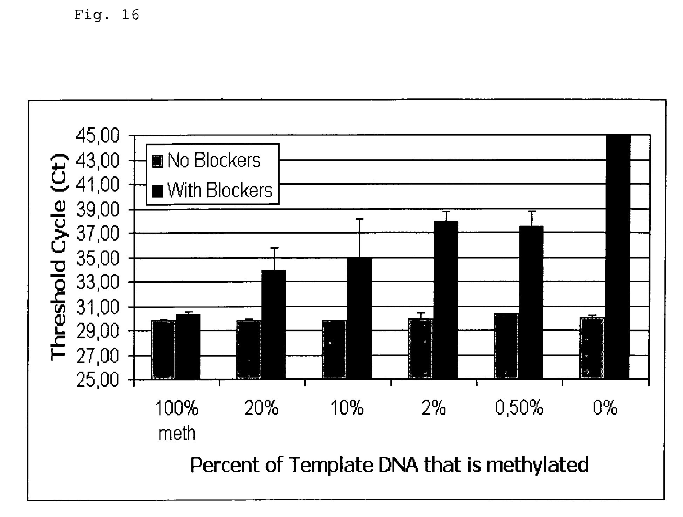

Zur Bestimmung der relativen Sensitivität wurden PCR von GSTp1 mit 10 ng der Templat-DNA-Mischungen (siehe Beispiel 8) mit und ohne Blocker-Oligonukleotide durchgeführt (Figur 16). Die Berechnung der "threshold cycle" erfolgte durch die TaqMan Software und gibt, vergleichbar zu den "crossing point"-Werten des Lightcycler, die Anzahl der PCR-Zyklen an, bei der das GSTP1 PCR-Produkt das erste Mal, mit einem höheren Signal als die Negativkontrolle, detektiert werden konnte. Dies bedeutet, je niedriger der "threshold cycle"-Wert, um so effizienter erfolgte die Amplifikation des GSTp1-Fragments.

Die ermittelten "threshold cycle"-Werte zeigten, dass in einer PCR mit Blocker-Oligonukleotid eine Kopie des methylierten GSTp1 Gen, in einem Hintergrund von 200 Kopien des unmethylierten GSTP1 Gens reproduzierbar detektiert werden kann (siehe Figur 16). Dies entspricht einer absoluten Sensitivität von 50 pg methylierter Templat-DNA. Unter den gleichen Bedingungen werden die Amplifikation des GSTp1-Gens von 10 ng unmethylierter Bisulfit-behandelter Templat-DNA komplett unterdrückt (siehe Figur 16).

### Beschreibung der Figuren

Figur 10: Agarose-Gel von GSTp1-PCR-Fragmenten. Die PCR wurde mit 10 ng, 5 ng, 1 ng, 0,5 ng und 0,1 ng methylierter (A) und unmethylierter (B), Bisulfit-behandelter Templat-DNA durchgeführt.
Figur 11: Sequenz des GSTp1-Fragments mit Lage der Primer und Blocker-Oligonukleotide.
Figur 12: Agarose-Gele von GSTp1-PCR-Fragmenten. Die relative Sensitivität der Amplifikation des methylierten GSTp1-Gens (A, B, D, E) und absolute Sensitivität (C, F) der Amplifikation des methylierten GSTp1-Gens wurden analysiert. Die GSTp1 PCR wurden mit Blocker-Oligonukleotid (A, B, C) und ohne Blocker-Oligonukleotid (D, E, F) durchgeführt. Als Templat-DNA wurde verwendet: 20 ng methylierte Bisulfit-behandelte DNA (A1, B1, D1, E1, C1, F1, C2, F2) und 20 ng unmethylierte Bisulfit-behandelte DNA (A8, B8, D8, E8); methylierte und unmethylierte Bisulfit-behandelte DNA im Mischungsverhältnis 1:2 (A2, B2, D2, E2), 1:10 (A3, B3, D3, E3), 1:20 (A4, B4, D4, E4), 1:100 (A5, B5, D5, E5), 1:200 (A6, B6, D6, E6), 1:1000 (A7, B7, D7, E7); 10 ng (C3, F3), 2 ng (C4, F4),1 ng (C5, F5),0,2 ng (C6, F6), 0,1 ng (C7, F7), 0,02 ng (C8, F8),methylierte Bisulfit-behandelte DNA und keine DNA (A9, D9) .
Figur 13: Analyse der Methylierungsspezifität der Detektionssonden. Die Figur zeigt den Fluoreszenzverlauf während der GSTp1-PCR von methylierter Bisulfit-behandelter DNA (durchgezogene Linie) und unmethylierter Bisulfit-behandelter DNA (gepunktete Linie), detektiert mit Hybridisierungssonde GSTp1-Red 650 (A) bzw. GSTp1-Red 705 (B).
Figur 14: Bestimmung der absoluten Sensitivität der GSTp1-PCR auf dem LightCycler. Die GSTp1 PCR wurden mit Blocker-Oligonukleotid ("rotor position" 9,10,11,12,13,14,15,16,18) und ohne Blocker-Oligonukleotid ("rotor position" 1,2,3,4,5,6,7,8,17)durchgeführt. Als Templat-DNA wurde verwendet: methylierte Bisulfit-behandelte DNA 7,5 ng ("rotor position" 1,9), 3,7 ng ("rotor position" 2, 10), 0,75 ng ("rotor position" 3,11),0,37 ng ("rotor position" 4,12), 0,075 ng ("rotor position" 5,13), 0,037 ng ("rotor position" 6,14), 0,015 ng ("rotor position" 7,15),0,0075 ng ("rotor position" 8,16) und keine DNA ("rotor position" 17, 18) .
Figur 15: Bestimmung der relativen Sensitivität der Amplifikation des methylierten GSTp1-Gens. Die Amplifikation des methylierten GSTp1-Gens wurden mit LightCycler durchgeführt. Die Detektion mit Hybridisierungssonde GSTp1-Red 650 (F2/F1, Crossing Point) und GSTp1-Red 705 (F3/F1, Crossing Point) ist angegeben. Die GSTp1 PCR wurden mit Blocker-Oligonukleotid ("rotor position" 11,12,13,14,15,17,18,19,20) und ohne Blocker-Oligonukleotid ("rotor position" 1,2,3,4,5,7,8,9,10) durchgeführt. Als Templat-DNA wurde verwendet: 15 ng methylierte Bisulfit-behandelte DNA ("rotor position" 1, 11) und 15 ng unmethylierte Bisulfit-behandelte DNA ("rotor position" 10, 20), .methylierte und unmethylierte Bisulfit-behandelte DNA im Mischungsverhältnis 1:2 ("rotor position" 2,12), 1:10 ("rotor position" 3, 13), 1:20 ("rotor position" 4,14), 1:100 ("rotor position" 5,15), 1:500 ("rotor position" 7,17), 1:1000 ("rotor position" 8,18), und keine DNA ("rotor position" 10,20).
Figur 16: Bestimmung der relativen Sensitivität der Amplifikation des methylierten GSTp1-Gens. Die Amplifikation des methylierten GSTp1-Gens wurden mit TaqMan durchgeführt.

## Patentansprüche

1. Verfahren zum Nachweis von Cytosin-Merthylierung in DNA-Proben, **dadurch gekennzeichnet, dass** man die folgenden Schritte ausführt:
man behandelt eine genomische DNA-Probe, welche zu untersuchende DNA und Hintergrund-DNA umfasst, chemisch derart, dass alle nicht methylierten Cytosinbasen in Uracil umgewandelt werden, während die 5-Methylcytosinbasen unverändert bleiben,
man amplifiziert die chemisch behandelte DNA-Probe unter Verwendung von mindestens 2 Primeroligonukleotiden,
sowie einer Polymerase, wobei die zu untersuchende DNA gegenüber der Hintergrund-DNA als Templat bevorzugt wird und wobei die Amplifikation nicht in der Gegenwart eines zusätzlichen Oligonukleotides oder eines PNA-Oligomers, welches bevorzugt an ein 5'-CA-3'-Dinukleotid oder an ein 5'-TG-3'-Dinukleotid oder an ein 5'-CA-3'-Dinukleotid der Hintergrund-DNA bindet und deren Amplifikation nachteilig beeinflusst und
man analysiert die Amplifikate und schließt aus der Analyse weiterer Positionen auf den Methylierungsstatus in der zu untersuchenden DNA,

2. Verfahren nach Anspruch 1, **dadurch gekennzeichnet, dass** man die Proben DNA aus Serum oder anderen Körperflüssigkeiten eines Individuums gewinnt.

3. Verfahren nach Anspruch 1, **dadurch gekennzeichnet, dass** man die Proben DNA aus Zelllinien, Blut, Sputum, Stuhl, Urin, Serum, Gehirn-Rückenmarks-Flüssigkeit, in Paraffin eingebettetem Gewebe, beispielsweise Gewebe von Augen, Darm, Niere, Hirn, Herz, Prostata, Lunge, Brust oder Leber, histologischen Objektträgern und allen möglichen Kombinationen hiervon gewinnt.

4. Verfahren nach einem der voranstehenden Ansprüche, **dadurch gekennzeichnet, dass** man die chemische Behandlung mit einem Bisulfit (= Disulfit, Hydrogensulfit) durchführt.

5. Verfahren nach Anspruch 4, **dadurch gekennzeichnet, dass** die chemische Behandlung nach Einbetten der DNA in Agarose erfolgt.

6. Verfahren nach Anspruch 4, **dadurch gekennzeichnet, dass** bei der chemischen Behandlung ein die DNA-Duplex denaturierendes Reagenz und/oder ein Radikalfänger zugegen ist.

7. Verfahren nach einem der voranstehenden Ansprüche, **dadurch gekennzeichnet, dass** man die chemisch behandelte DNA-Probe im zweiten Schritt unter Verwendung von mindestens 2 Primeroligonukleotiden und mindestens einem Reporteroligonukleotid, welches an ein 5'-CG-3'-Dinukleotid oder ein 5'-TG-3'-Dinukleotid oder ein 5'-CA-3'-Dinukleotid hybridisiert, sowie einer Polymerase amplifiziert, wobei das Reporteroligonukleotid bevorzugt an die zu untersuchende DNA bindet und deren Amplifikation anzeigt.

8. Verfahren nach Anspruch 7, **dadurch gekennzeichnet, dass** man zusätzlich zu dem Reporteroligonukleotid ein weiteres mit einem Fluoreszenzfarbstoff markiertes Oligomer verwendet, welches unmittelbar benachbart zu dem Reporteroligonukleotid hybridisiert und sich diese Hybridisierung mittels Fluoreszenz Resonanz Energietransfer nachweisen lässt.

9. Verfahren nach einem der Ansprüche 7 oder 9, **dadurch gekennzeichnet, dass** ein Taqman-Assay durchgeführt wird.

10. Verfahren nach einem der Ansprüche 7 oder 9, **dadurch gekennzeichnet, dass** ein Lightcycler Assay durchgeführt wird.

11. Verfahren nach einem der Ansprüche 7 bis 10, **dadurch gekennzeichnet, dass** die Reporteroligonukleotide mindestens eine Fluoreszenzmarkierung tragen.

12. Verfahren nach einem der Ansprüche 7 bis 11, **dadurch gekennzeichnet, dass** die Reportermoleküle die Amplifikation entweder durch eine Zunahme oder eine Abnahme der Fluoreszenz anzeigen.

13. Verfahren nach Anspruch 12, **dadurch gekennzeichnet, dass** man die Zunahme oder Abnahme der Fluoreszenz auch direkt zur Analyse verwendet und aus dem Fluoreszenzsignal auf einen Methylierungszustand der zu analysierenden DNA schließt.

14. Verfahren nach einem der voranstehenden Ansprüche, **dadurch gekennzeichnet, dass** die Hintergrund-DNA in 100facher Konzentration im Vergleich zur zu untersuchenden DNA vorliegt.

15. Verfahren nach einem der voranstehenden Ansprüche, **dadurch gekennzeichnet, dass** die Hintergrund-DNA in 1000facher Konzentration im Vergleich zur zu untersuchenden DNA vorliegt.

16. Verfahren nach einem der voranstehenden Ansprüche, **dadurch gekennzeichnet, dass** die Analyse mittels Hybridisierung an Oligomer-Arrays erfolgt, wobei Oligomere Nukleinsäuren oder in ihren Hybridisierungseigenschaften ähnliche Moleküle wie PNAs sein können.

17. Verfahren nach Anspruch 16, **dadurch gekennzeichnet, dass** die Oligomere über einen 12-22 Basen langen Abschnitt an die zu analysierende DNA hybridisieren und sie ein CG, TG oder CA Dinukleotid umfassen.

18. Verfahren nach einem der Ansprüche 16 oder 17, **dadurch gekennzeichnet, dass** der Methylierungsstatus von mehr als 20 Methylierungspositionen der zu analysierenden DNA in einem Experiment nachgewiesen wird.

19. Verfahren nach einem der Ansprüche 16 oder 17, **dadurch gekennzeichnet, dass** der Methylierungsstatus von mehr als 60 Methylierungspositionen der zu analysierenden DNA in einem Experiment nachgewiesen wird.

20. Verfahren nach einem der Ansprüche 1 bis 15, **dadurch gekennzeichnet, dass** die Analyse durch Längenmessung der amplifizierten zu untersuchenden DNA erfolgt, wobei Methoden zur Längenmessung Gelelektrophorese, Kapillargelelektrophorese, Chromatographie (z.B. HPLC), Massenspektrometrie und andere geeignete Methoden umfassen.

21. Verfahren nach einem der Ansprüche 1 bis 15, **dadurch gekennzeichnet, dass** die Analyse durch Sequenzierung erfolgt, wobei Methoden zur Sequenzierung die Sanger-Methode, Maxam-Gilbert-Methode und andere Methoden wie Sequencing by Hybridisation (SBH) umfassen.

22. Verfahren nach Anspruch 21, **dadurch gekennzeichnet, dass** man die Sequenzierung für jede oder eine kleine Gruppe von CpG Positionen mit jeweils einem separaten Primeroligonukleotid ausführt und die Verlängerung der Primer nur eine oder einige wenige Basen ausmacht, und man aus der Art der Primerverlängerung auf den Methylierungsstatus der betreffenden Positionen in der zu untersuchenden DNA schließt.

23. Verfahren nach einem der voranstehenden Ansprüche, **dadurch gekennzeichnet, dass** man aus dem Methylierungsgrad an den verschiedenen untersuchten CpG Positionen auf das Vorliegen einer Erkrankung oder eines anderen medizinischen Zustandes des Patienten schließt.

24. Verfahren nach einem der voranstehenden Ansprüche, **dadurch gekennzeichnet, dass** die Amplifikate selbst für die Detektion mit einer nachweisbaren Markierung versehen sind.

25. Verfahren nach Anspruch 24, **dadurch gekennzeichnet, dass** die Markierungen Fluoreszenzmarkierungen sind.

26. Verfahren nach Anspruch 24, **dadurch gekennzeichnet, dass** die Markierungen Radionuklide sind.

27. Verfahren nach Anspruch 24, **dadurch gekennzeichnet, dass** die Markierungen ablösbare Massenmarkierungen sind, die in einem Massenspektrometer nachgewiesen werden.

28. Verfahren nach einem der voranstehenden Ansprüche, **dadurch gekennzeichnet, dass** bei der Amplifikation einer der Primer an eine Festphase gebunden ist.

29. Verfahren nach einem der Ansprüche 1 bis 24, **dadurch gekennzeichnet, dass** die Amplifikate insgesamt im Massenspektrometer nachgewiesen werden und somit durch ihre Masse eindeutig charakterisiert sind.

30. Verwendung eines Verfahrens nach einem der voranstehenden Ansprüche zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

31. Verwendung eines Verfahrens nach einem der voranstehenden Ansprüche zur Unterscheidung von Zelltypen oder Geweben oder zur Untersuchung der Zelldifferenzierung.

32. Kit, bestehend aus einem Bisulfit enthaltenen Reagenz, Primern und weiteren Oligonukleotiden ohne 3'-OH Funktion zur Herstellung der Amplifikate, sowie optional einer Anleitung zur Durchführung eines Assays nach einem der Ansprüche 1-29.
